# EUROPEAN PATENT SPECIFICATION

(11) **EP 1 675 832 B1**
(45) Date of publication and mention of the grant of the patent: **20.02.2008**
(21) Application number: 04790757.1
(22) Date of filing: 21.10.2004
(51) Int. Cl.: C07D 231/14, C07D 401/12, C07D 409/04, C07D 207/34, A61K 31/415, A61K 31/4439, A61K 31/4155, A61K 31/40

(54) **HETEROCYCLYL COMPOUNDS**
HETEROCYCLYLVERBINDUNGEN
COMPOSES HETEROCYCLYLES

(30) Priority: 24.10.2003 GB 0324893; 24.10.2003 GB 0324895
(43) Date of publication of application: 05.07.2006
(73) Proprietor: GLAXO GROUP LIMITED, Greenford, Middlesex UB6 ONN (GB)
(72) Inventor: GIBLIN, Gerard, Martin, Paul, GlaxoSmithKline, Harlow Essex CM19 5AW (GB); HALL, Adrian, GlaxoSmithKline, Harlow Essex CM19 5AW (GB); HURST, David, Nigel, GlaxoSmithKline, Harlow Essex CM19 5AW (GB); LEWELL, Xiao, Qing, GlaxoSmithKline, Stevenage Hertfordshire SG1 2NY (GB); LORTHIOIR, Olivier, Eric, GlaxoSmithKline, Stevenage Hertfordshire SG1 2NY (GB); MCKEOWN, Stephen, Carl, GlaxoSmithKline, Harlow Essex CM19 5AW (GB); SCOCCITTI, Tiziana, GlaxoSmithKline, Harlow Essex CM19 5AW (GB); WATSON, Stephen, Paul, GlaxoSmithKline, Stevenage Hertfordshire SG1 2NY (GB)
(74) Representative: Stevenson, Dian Elizabeth
(86) International application number: PCT/EP2004/011964
(87) International publication number: WO 2005/040128

(56) References cited:
- EP-A- 0 752 421
- WO-A-01/19814
- WO-A-03/048137
- WO-A-03/084917
- WO-A-03/088908
- WO-A-03/101959
- WO-A-20/04039753
- WO-A-20/04060870
- WO-A-20/04060888
- WO-A-20/04083185
- LOUBINOUX, BERNARD ET AL: "Preparation of 1-aryloxymethyl-1,2,3-triazoles" JOURNAL OF HETEROCYCLIC CHEMISTRY , 21(6), 1669-72 CODEN: JHTCAD; ISSN: 0022-152X, 1984, XP002313523
- DATABASE CHEMCATS CHEMICAL ABSTRACTS SERVICE, COLUMBUS, OHIO, US; XP002313524 & 9 May 2003 (2003-05-09), INTERBIOSCREEN LTD, PO BOX 218, MOSCOW, 121019
- DATABASE CHEMCATS CHEMICAL ABSTRACTS SERVICE, COLUMBUS, OHIO, US; XP002313525 & 17 September 2004 (2004-09-17), INTERCHIM, 213 AVENUE KENNEDY, BP 1140, MONTLUCON, CEDEX,03103,FRANCE

## Description

This invention relates to heterocyclic compounds, to processes for their preparation, to pharmaceutical compositions containing them and to their use in medicine, in particular their use in the treatment of conditions mediated by the action of PGE₂ at the EP₁ receptor and conditions mediated by the action of thromboxane on the TP receptor. The invention also relates to compounds having activity at both the EP₁ and TP receptors.

The EP₁ receptor is a 7-transmembrane receptor and its natural ligand is the prostaglandin PGE₂. PGE₂ also has affinity for the other EP receptors (types EP₂, EP₃ and EP₄). The EP₁ receptor is associated with smooth muscle contraction, pain (in particular inflammatory, neuropathic and visceral), inflammation, allergic activities, renal regulation and gastric or enteric mucus secretion. We have now found a novel group of compounds which bind with high affinity to the EP₁ receptor.

A number of review articles describe the characterization and therapeutic relevance of the prostanoid receptors as well as the most commonly used selective agonists and antagonists: Eicosanoids; From Biotechnology to Therapeutic Applications, Folco, Samuelsson, Maclouf, and Velo eds, Plenum Press, New York, 1996, chap. 14, 137-154 and Journal of Lipid Mediators and Cell Signalling, 1996, 14, 83-87 and Prostanoid Receptors, Structure, Properties and Function, S Narumiya et al, Physiological Reviews 1999, 79(4), 1193-126. An article from The British Journal of Pharmacology, 1994, 112, 735- 740 suggests that Prostaglandin E₂ (PGE₂) exerts allodynia through the EP₁ receptor subtype and hyperalgesia through EP₂ and EP₃ receptors in the mouse spinal cord. Furthermore an article from *The Journal of Clinical Investigation,* 2001, 107 (3), 325 shows that in the EP₁ knock-out mouse pain-sensitivity responses are reduced by approximately 50%. Two papers from *Anesthesia and Analgesia* have shown that (2001, 93, 1012-7) an EP₁ receptor antagonist (ONO-8711) reduces hyperalgesia and allodynia in a rat model of chronic constriction injury, and that (2001, 92, 233-238) the same antagonist inhibits mechanical hyperalgesia in a rodent model of post-operative pain. S. Sarkar et al in Gastroenterology, 2003, 124(1), 18-25 demonstrate the efficacy of EP₁ receptor antagonists in the treatment of visceral pain in a human model of hypersensitivity. Thus, selective prostaglandin ligands, agonists or antagonists, depending on which prostaglandin E receptor subtype is being considered, have anti-inflammatory, antipyretic and analgesic properties similar to a conventional non-steroidal anti-inflammatory drug, and in addition, inhibit hormone-induced uterine contractions and have anti-cancer effects. These compounds have a diminished ability to induce some of the mechanism-based side effects of NSAIDs which are indiscriminate cyclooxygenase inhibitors. In particular, the compounds have a reduced potential for gastrointestinal toxicity, a reduced potential for renal side effects, a reduced effect on bleeding times and a lessened ability to induce asthma attacks in aspirin-sensitive asthmatic subjects. Moreover, by sparing potentially beneficial prostaglandin pathways, these agents may have enhanced efficacy over NSAIDS and/or COX-2 inhibitors.

Certain compounds of the present invention also exhibit antagonism at the TP receptor.

The TP (also known as TxA₂) receptor is a prostanoid receptor subtype stimulated by the endogenous mediator thromboxane. Activation of this receptor results in various physiological actions primarily incurred by its platelet aggregatory and smooth muscle constricting effects, thus opposing those of prostacyclin receptor activation.

TP receptors have been identified in human kidneys (G.P. Brown et al, Prostaglandins and otherlipid mediators,1999, 57, 179-188) in the glomerulus and extraglomerular vascular tissue. Activation of TP receptors constricts glomerular capillaries and suppresses glomerular filtration rates (M.D. Breyer et al, Current Opinion in Nephrology and Hypertension, 2000, 9, 23-29), indicating that TP receptor antagonists could be useful for renal dysfunction in glomerulonephritis, diabetes mellitus and sepsis.

Activation of TP receptors induces bronchoconstriction, increase in microvascular permeability, formation of mucosal oedema and mucus secretion, typical characteristic features of bronchial asthma (T. Obata et al, Clinical Review of Allergy, 1994, 12(1), 79-93). TP antagonists have been investigated as potential asthma treatments resulting in, for example, orally active Seratrodast (AA-2414) (S. Terao et al, Yakugaku Zasshi, 1999, 119(5), 377-390). Ramatroban is another TP receptor antagonist currently undergoing phase III clinical trials as an anti-asthmatic compound.

Antagonists at the TP receptor have been shown to have a gastroprotective effect. In rats it has been shown that SQ 33961 and BM 13505 inhibit gastric lesions induced by taurocholate acid, aspirin or indomethacin (E.H. Ogletree et al, Journal of Pharmacology and Experimental Therapeutics, 192, 263(1), 374-380.

In The American Physiological Society (1994, 267, R289-R-294), studies suggest that PGE₂-induced hyperthermia in the rat is mediated predominantly through the EP₁ receptor.

WO 96/06822 (March 7, 1996), WO 96/11902 (April 25, 1996), EP 752421-A1 (January 08, 1997), WO 01/19814 (22 March 2001), WO 03/084917 (16 October 2003), WO 03/101959 (11 December 2003), WO 2004/039753 (13 May 2004) and WO2004/083185 (30 September 2004) disclose compounds as being useful in the treatment of prostaglandin mediated diseases.

P. Lacombe et al (220th National Meeting of The American Chemical Society, Washington D.C., USA, 20-24 August, 2000) disclosed 2,3-diarylthiophenes as ligands for the human EP₁ prostanoid receptor. Y. Ducharme et al (18th International Symposium on Medicinal Chemistry; Copenhagen, Denmark and Malmo, Sweden; 15th-19th August 2004) disclosed 2,3-diarylthiophenes as EP₁ receptor antagonists.

WO 03/048137 (published 12 June 2003) discloses certain phenyl compounds as mGluR5 modulators useful in the treatment of psychiatric and mood disorders such as schizophrenia, anxiety, depression and panic, as well as in the treatment of pain and other diseases.

B. Loubinoux et al, J. Heterocyclic Chem, 1984, 21(6) 1669-72 relates to the preparation of 1-aryloxymethyl-1,2,3-triazoles.

Chemcats Database Chemical Abstracts Service, Columbus, Ohio, (9 May 2003) discloses certain 1 H-pyrrole-3-carboxylic esters as part of a chemical library.

WO 03/088908 (published 30 October 2003) discloses heterocyclic compounds useful as inhibitors of potassium channel function, methods of using such compounds in the prevention and treatment of arrhythmia and IKur-associated conditions, and pharmaceutical compositions containing such compounds.

WO 04/060870 (published 22 July 2004) discloses certain CB1 receptor inverse agonists useful for the treatment and/or prophylaxis of diseases associated with the modulation of CB1 receptors.

WO 04/060888 (published 22 July 2004) discloses certain CB1 receptor inverse agonists useful for the treatment and/or prophylaxis of diseases associated with the modulation of CB1 receptors.

It is now suggested that a novel group of pyrazole derivatives surprisingly are selective for the EP₁ receptor over the EP₃ receptor, and are therefore indicated to be useful in treating conditions mediated by the action of PGE₂ at EP₁ receptors. Such conditions include pain, or inflammatory, immunological, bone, neurodegenerative or renal disorders.

It is also suggested that this novel group of pyrazole derivatives are antagonists at the TP receptor and are therefore indicated to be useful in treating conditions mediated by the action of thromboxane at the TP receptor. Such conditions include those disclosed in WO 2004/039807 (Merck Frosst Canada & Co) which is incorporated herein by reference, and include respiratory diseases e.g. asthma, allergic diseases, male erectile dysfunction, thrombosis, renal disorders and gastric lesions.

Accordingly the present invention provides compounds of formula (1): 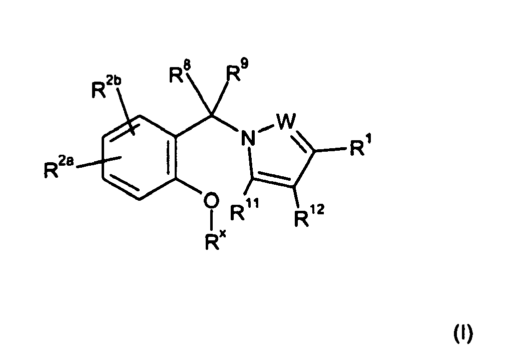 wherein:
W is N or CR¹⁰;
R¹ is CO₂H;
R^{2a} and R^{2b} are independently selected from hydrogen, halo, C₁₋₄alkyl, CF₃, and OC₁₋₆alkyl;
R^{x} is selected from CH₂-pyridyl, C₁₋₆alkyl or CH₂Ph wherein Ph is substituted by R^{3a}, R^{3b} and R^{3c};
R^{3a}, R^{3b} and R^{3c} are independently selected from hydrogen, halogen, NO₂, OCH₃, CH₃ and CF₃;
R⁸ is hydrogen;
R⁹ is hydrogen or CH₃;
R¹⁰ is selected from hydrogen, halogen, CH₃ and CF₃;
R¹¹ is selected from hydrogen, halogen, CH₃, CF₃ and thienyl; and
R¹² is selected from hydrogen, halogen, CH₃ and CF₃;
or a pharmaceutically acceptable salt or solvate thereof.

Compounds of formula (I) include the compounds of examples 1 to 61 and pharmaceutically acceptable salts or solvates thereof.

The compounds of the invention are selective for EP₁ over EP₃. Preferred compounds are 100 fold selective for EP₁ over EP₃.

The invention is described using the following definitions unless otherwise indicated.

Pharmaceutically acceptable salts include those described by Berge, Bighley and Monkhouse, J. Pharm. Sci., 1977, 66, 1-19. The term "pharmaceutically acceptable salts" refers to salts prepared from pharmaceutically acceptable bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic bases include salts of primary, secondary, and tertiary amines; substituted amines including naturally occurring substituted amines; and cyclic amines. Pharmaceutically acceptable organic bases include arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, procaine, purines, theobromine, triethylamine, trimethylamine, tripropyl amine, tromethamine, and the like. Salts may also be formed from basic ion exchange resins, for example polyamine resins. When the compound of the present invention is basic, salts may be prepared from pharmaceutically acceptable acids, including inorganic and organic acids. Such acids include acetic, benzenesulfonic, benzoic, camphorsulfonic, citric, ethanesulfonic, ethanedisulfonic, fumaric, gluconic, glutamic, hydrobromic, hydrochloric, isethionic, lactic, maleic, malic, mandelic, methanesulfonic, mucic, pamoic, pantothenic, phosphoric, propionic, succinic, sulfuric, tartaric, p-toluenesulfonic acid, and the like.

The compounds of formula (I) may be prepared in crystalline or non-crystalline form, and if crystalline, may be optionally hydrated or solvated. This invention includes in its scope stoichiometric hydrates as well as compounds containing variable amounts of water.

Suitable solvates include pharmaceutically acceptable solvates, such as hydrates.

Solvates include stoichiometric solvates and non-stoichiometric solvates.

The terms "halogen" or "halo" are used to represent fluorine, chlorine, bromine or iodine.

The term "alkyl" as a group or part of a group means a straight, branched or cyclic chain alkyl group or combinations thereof. Unless hereinbefore defined, examples of alkyl include C₁₋₈alkyl, for example methyl, ethyl, n-propyl, iso-propyl, n-butyl, sec-butyl, isobutyl, t-butyl, pentyl, hexyl, 1,1-dimethylethyl, cyclopentyl or cyclohexyl or combinations thereof such as cyclohexylmethyl and cyclopentylmethyl.

The term "alkoxy" as a group or as part of a group means a straight, branched or cyclic chain alkoxy group. Unless hereinbefore defined "alkoxy" includes C₁₋₈alkoxy, e.g. methoxy, ethoxy, n-propoxy, iso-propoxy, n-butoxy, sec-butoxy,iso-butoxy, t-butoxy, pentoxy, hexyloxy, cyclopentoxy or cyclohexyloxy. In one aspect "alkoxy" is C₁₋₆ alkoxy.

The term "heterocyclyl" as a group or as part of a group means an aromatic or nonaromatic five or six membered ring which contains from 1 to 4 heteroatoms selected from nitrogen, oxygen or sulfur and is unsubstituted or substituted by, for example, up to three substituents, preferably one or two substituents. Examples of 5- membered heterocyclyl groups include furyl, dioxalanyl, pyrrolyl, oxazolyl, thiazolyl, imidazolyl, oxadiazolyl, thiadiazolyl, triazolyl, triazinyl, isothiazolyl, isoxazolyl, thiophenyl, pyrazolyl or tetrazolyl. Examples of 6-membered heterocyclyl groups are pyridyl, pyridazinyl, pyrimidinyl, pyrazinyl, triazinyl or tetrazinyl.

The term "aryl" as a group or part of a group means a 5- or 6- membered aromatic ring, for example phenyl, or a 7 to 12 membered bicyclic ring system where at least one of the rings is aromatic, for example naphthyl. An aryl group may be optionally substituted by one or more substituents, for example up to 4, 3 or 2 substituents. Preferably the aryl group is phenyl.

The term "heteroaryl" as a group or as part of a group means a monocyclic five or six membered aromatic ring, or a fused bicyclic aromatic ring system comprising two of such monocyclic five or six membered aromatic rings. These heteroaryl rings contain one or more heteroatoms selected from nitrogen, oxygen or sulfur, where N-oxides, sulfur oxides and sulfur dioxides are permissible heteroatom substitutions. A heteroaryl group may be optionally substituted by one or more substituents, for example up to 3 or up to 2 substituents. Examples of "heteroaryl" include furyl, thienyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl, pyrimidinyl, quinolinyl, isoquinolinyl, benzofuryl, benzothienyl, indolyl, and indazolyl.

The term "bicyclic heterocyclyl" when used herein means a fused bicyclic aromatic or nonaromatic bicyclic heterocyclyl ring system comprising up to four, preferably one or two, heteroatoms each selected from oxygen, nitrogen and sulphur. Each ring may have from 4 to 7, preferably 5 or 6, ring atoms. A bicyclic heteroaromatic ring system may include a carbocyclic ring. Examples of bicyclic heterocyclyl groups include quinolinyl, isoquinolinyl, quinoxalinyl, quinazolinyl, pyridopyrazinyl, benzoxazolyl, benzothiophenyl, benzimidazolyl, benzothiazolyl, benzoxadiazolyl, benzthiadiazolyl, indolyl, benztriazolyl or naphthyridinyl.

When the heteroatom nitrogen replaces a carbon atom in an alkyl group, or when nitrogen is present in a heteroaryl, heterocyclyl or bicyclic heterocyclyl group, the nitrogen atom will, where appropriate be substituted by one or two substituents selected from hydrogen and C₁₋₈alkyl, preferably hydrogen and C₁₋₆alkyl, more preferably hydrogen.

Optional substituents for alkyl groups unless hereinbefore defined include OH, CO₂H, CO₂C₁-₆alkyl, NHC₁₋₆alkyl, NH₂, (O), OC₁₋₆alkyl, phenyl or halo e.g. Cl, Br or F. An alkyl group may be substituted by one or more optional substituents, for example up to 5, 4, 3, 2 or 1 optional substituents. In one aspect substituted alkyl groups include those substituted by one or more fluorine atoms, up to per-fluorination, e.g. CF₃.

Optional substituents for alkoxy groups unless hereinbefore defined include OH, and halo e.g. Cl, Br or F. An alkoxy group may be substituted by one or more optional substituents, for example up to 5, 4, 3, or 2 optional substituents.

Unless otherwise defined, optional substituents for aryl, heteroaryl or heterocyclyl moieties as a group or part of a group are selected from C₁₋₆alkyl, C₁₋₆alkoxy and halogen.

Compounds of formula (I) can be prepared as set forth in the following schemes and in the examples. The following processes form another aspect of the present invention.

For example, compounds of formula (I)' may be prepared by the general route below: 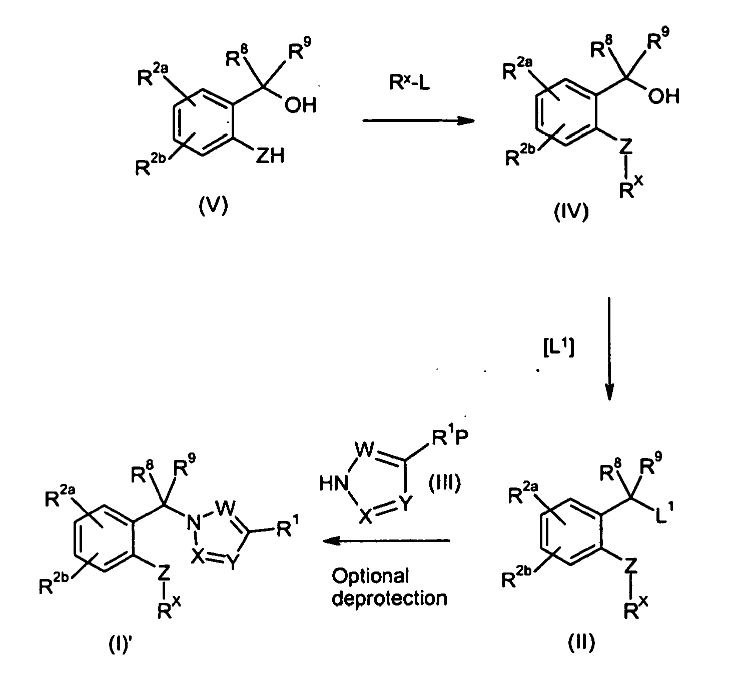 wherein L and L¹ are leaving groups, for example halo e.g. bromo; and W, X, Y, Z, R^{2a}, R^{2b}, R¹, R⁸, R⁹, and R^{x} are as defined below for compounds of formula (I)', and P is an optional protecting group. The skilled person will recognise when the use of a protecting group is necessary. When R¹ is CO₂H, R¹P is suitably CO₂C₁₋₄alkyl or optionally substituted benzyl.

Suitable reaction conditions for the reaction of an azole of formula (III) with a compound of formula (II) to give a compound of formula (I)' include heating in a solvent, e.g. ethanol, in the presence of a base, e.g. potassium *tert*-butoxide.

Suitable reaction conditions for the preparation a compound of formula (II) include conventional methods for converting the hydroxy group of the compound of formula (IV) to a leaving group, for example when L¹ is Br, the compound of formula (IV) may be reacted with phosporous tribromide in a solvent, e.g. dichloromethane, at reduced temperatures, e.g. less than -10°C.

Suitable reaction conditions for the reaction of a compound of formula (V) with a compound R^{x}-L to give a compound of formula (IV) are known to those skilled in the art and include the use of a solvent e.g. a C₁₋₄alcohol such as methanol or ethanol in the presence of a base, e.g. sodium hydroxide. The skilled person will appreciate that when Z is SO or SO₂, the alkylation step is carried out when Z is S, and the sulfur is then oxidised to the required oxidation state by conventional means at an appropriate stage in the synthesis.

Accordingly the present invention also provides a process for the preparation of a compound of formula (I)' or a derivative thereof: 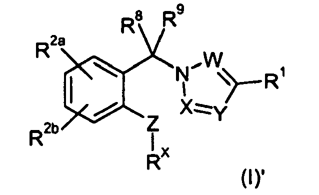 wherein:
W represents N or CR¹⁰ wherein R¹⁰ represents hydrogen, halogen, optionally substituted alkyl, optionally substituted aryl, or optionally substituted heterocyclyl;
X represents N or CR¹¹ wherein R¹¹ represents hydrogen, halogen, optionally substituted alkyl, optionally substituted aryl, or optionally substituted heterocyclyl;
Y represents N or CR¹² wherein R¹² represents hydrogen, halogen, CH₃ or CF₃;
Z represents O, S, SO or SO₂;
R¹ represents CO₂R⁴, CONR⁵R⁶, CH₂CO₂H, optionally substituted SO₂alkyl, SO₂NR⁵R⁶, NR⁵CONR⁵R⁶, 2H-tetrazol-5-yl-methyl or optionally substituted heterocyclyl;
R^{2a} and R^{2b} independently represents hydrogen, halo, optionally substituted alkyl, optionally substituted alkoxy, CN, SO₂alkyl, SR⁵, NO₂, optionally substituted aryl, CONR⁵R⁶ or optionally substituted heteroaryl;
R^{x} represents optionally substituted alkyl wherein 1 or 2 of the non-terminal carbon atoms are optionally substituted by a group independently selected from NR⁴, O and SOₙ,
wherein n is 0, 1 or 2: or R^{x} represents optionally substituted CQ^{a}Q^{b}-heterocyclyl, optionally substituted CQ^{a}Q^{b}-bicyclic heterocyclyl or optionally substituted CQ^{a}Q^{b}-aryl;
R⁴ represents hydrogen or an optionally substituted alkyl;
R⁵ represents hydrogen or an optionally substituted alkyl;
R⁶ represents hydrogen or optionally substituted alkyl, optionally substituted heteroaryl, optionally substituted SO₂aryl, optionally substituted SO₂alkyl, optionally substituted SO₂heteroaryl, CN, optionally substituted CQ^{a}O^{b}aryl, optionally substituted CQ^{a}Q^{b}heteroaryl or COR⁷;
R⁷ represents hydrogen, optionally substituted alkyl, optionally substituted heteroaryl or optionally substituted aryl;
R⁸ and R⁹ are independently selected from hydrogen, fluorine or alkyl, or R⁸ and R⁹ together with the carbon to which they are attached form a cycloalkyl ring, optionally containing up to one heteroatom selected from O, S, NH or N-alkyl;
wherein Q^{a} and Q^{b} are each independently selected from hydrogen, CH₃ and fluorine; comprising:
reacting a compound of formula (II): 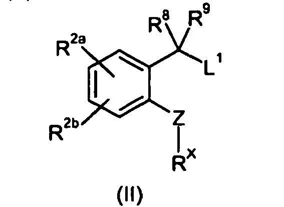
wherein L¹ is a leaving group and Z, R⁸. R⁹, R^{2a}, R^{2b}, and R^{x} are as defined above for a compound of formula (I);
with a compound of formula (III): 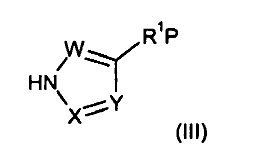 wherein W, X, Y, and R¹ are as defined above for a compound of formula (I) and P is an optional protecting group;
and where required, and in any order:
interconverting one substituent to another substituent; and/or
if necessary removing the optional protecting group; and/or
forming a derivative thereof.

Compounds of formula (I)' wherein Z is O, W is N, X is CR¹¹, Y is CR¹², and R¹ is COOH may be prepared by the general route below: 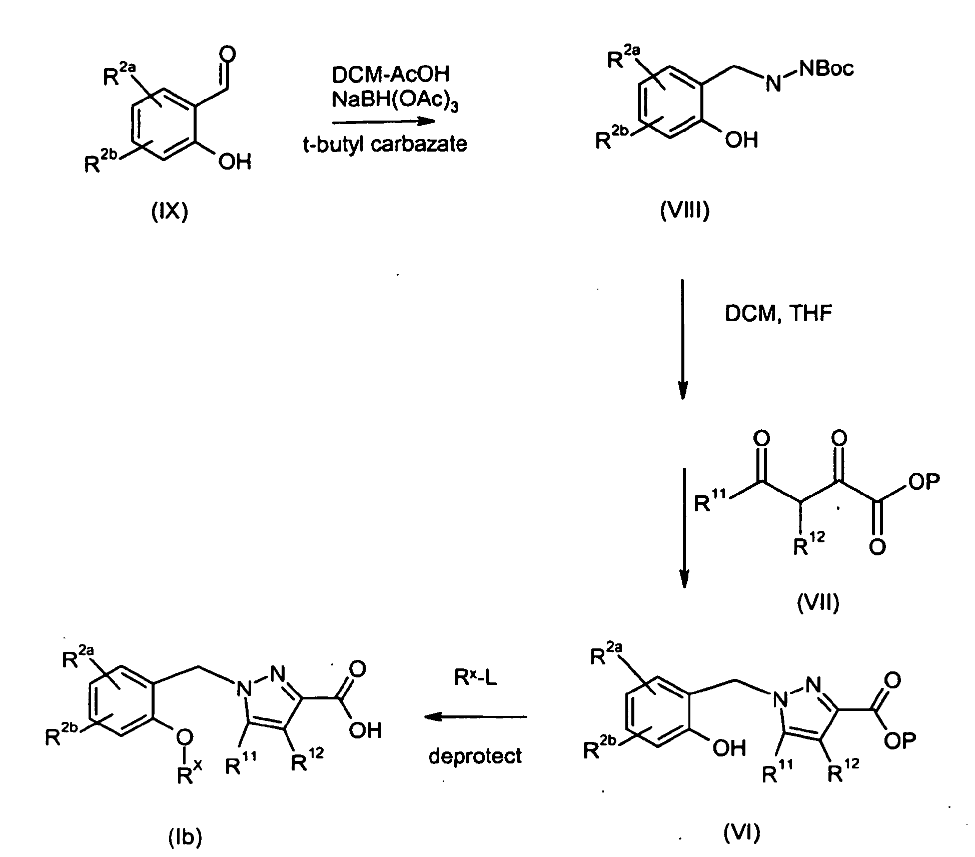 wherein L is a leaving group for example halo, e.g. bromo; P is a protecting group for example C₁₋₄ alkyl e.g. methyl or ethyl; and R^{2a}, R^{2b}, R¹¹, R¹² and R^{x} are as defined for compounds of formula (I).

Accordingly the present invention also provides a process for the preparation of a compound of formula (Ib) or a derivative thereof: 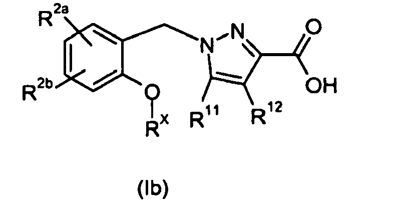 wherein:
R^{2a} and R^{2b} independently represents hydrogen, halo, optionally substituted alkyl, optionally substituted alkoxy, CN, SO₂alkyl, SR⁵, NO₂, optionally substituted aryl, CONR⁵R⁶ or optionally substituted heteroaryl;
R^{x} represents optionally substituted alkyl wherein 1 or 2 of the non-terminal carbon atoms are optionally substituted by a group independently selected from NR⁴, O and SOₙ, wherein n is 0, 1 or 2: or R^{x} may be optionally substituted CQ^{a}Q^{b}-heterocyclyl, optionally substituted CQ^{a}Q^{b}-bicyclic heterocyclyl or optionally substituted CQ^{a}Q^{b}-aryl;
R⁴ represents hydrogen or an optionally substituted alkyl;
R⁵ represents hydrogen or an optionally substituted alkyl;
R⁶ represents hydrogen or optionally substituted alkyl, optionally substituted heteroaryl, optionally substituted SO₂aryl, optionally substituted SO₂alkyl, optionally substituted SO₂heteroaryl, CN, optionally substituted CQ^{a}Q^{b}aryl, optionally substituted CQ^{a}Q^{b}heteroaryl or COR⁷;
R⁷ represents hydrogen, optionally substituted alkyl, optionally substituted heteroaryl or optionally substituted aryl;
R¹¹ represents hydrogen, halogen, optionally substituted alkyl, optionally substituted aryl, or optionally substituted heterocyclyl; and
R¹² represents hydrogen, halogen, CH₃ or CF₃;
wherein Q^{a} and Q^{b} are each independently selected from hydrogen, CH₃ and fluorine;
comprising:
reacting a compound of formula (VI): 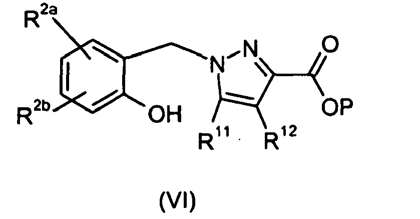
wherein R^{2a}, R^{2b}, R¹¹ and R¹² are as defined above for a compound of formula (Ib) and P is a protecting group;
with R^{x} - L wherein R^{x} is as defined for compounds of formula (I) and L is a leaving group; and where required, and in any order:
interconverting one substituent to another substituent; and/or
removing the protecting group; and/or
forming a derivative thereof.

When one or both of R¹¹ and R¹² is/are halogen, preferably the halogen group is introduced after the ring forming reaction of a compound of formula (VII) and (VIII).

Suitable fluorination conditions are described in e.g. K. Makino et al, J. Fluor. Chem, 1988, 39, 435-440. Halogenation conditions are also reviewed in e.g. Comprehensive heterocyclic chemistry. The structure, reactions, synthesis and uses of heterocyclic compounds, A.R. Katritzky and C.W. Rees (Eds), vols 1-8, Pergamon Press, Oxford, 1984; Comprehensive organic chemistry II. A review of the literature 1982-1995, A.R. Katritzky, C.W. Rees, and E.F.V. Scriven (eds), vols 1-11, Pergamon Press, Oxford, 1996, and Heterocyclic Chemistry, 4th Edition, J.A. Joule and K. Mills, Blackwell Science, 2000.

Suitable reaction conditions for the reaction of a compound of formula (VI) with a compound R^{x}-L are known to those skilled in the art and include the use of a solvent e.g. a C₁₋₄alcohol such as methanol or ethanol in the presence of a base, e.g. sodium hydroxide. Suitable conditions for the deprotection of an ester to give the corresponding carboxylic acid are known to those skilled in the art.

Suitable reaction conditions for the reaction of a compound of formula (VII) with a compound of formula (VIII) to give a pyrazole of formula (VI) will be apparent to the skilled person and include treatment with trifluoroacetic acid in a solvent, e.g. dichloromethane, at room temperature to remove the protecting group on the compound of formula (VIII) followed by condensation with (VII) in a solvent such as acetic acid or an alcohol such as methanol.

Suitable reaction conditions for the conversion of a salicylaldehyde of formula (IX) to a compound of formula (VIII) include reacting the salicylaldehyde with *tert*-butyl carbazate in the presence of acetic acid and sodium triacetoxyborohydride in a solvent such as dichloromethane.

Compounds of formula (I) wherein Z is O, W is CR¹⁰, X is CR¹¹, Y is CR¹², and R¹ is COOH may be prepared by the general route below: 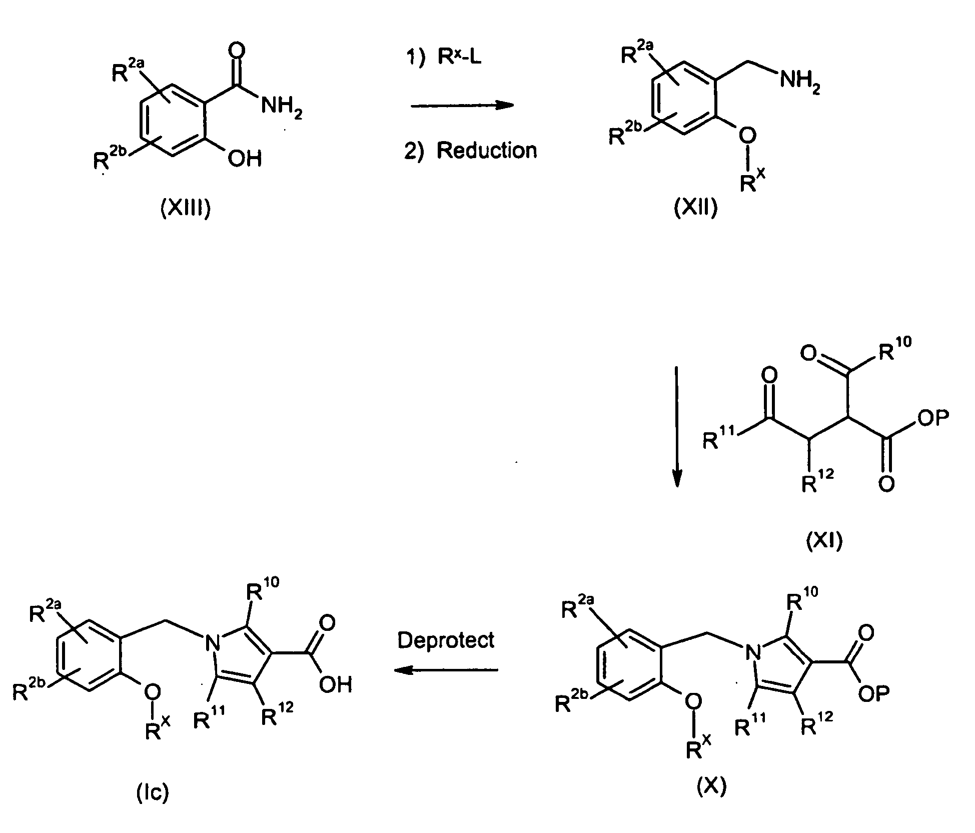 wherein L is a leaving group for example halo, e.g. bromo; P is a protecting group for example C₁₋₄ alkyl e.g. methyl or ethyl; and R^{2a}, R^{2b}, R¹⁰, R¹¹, R¹², and R^{x} are as defined for compounds of formula (Ia).

The skilled person will appreciate that one substituent R^{x} can be converted to a different substituent R^{x} by conventional means, as described, for example, in the methods of the Examples, at a suitable point during the synthesis.

Suitable reaction conditions for the reaction of a compound of formula (XIII) with a compound R^{x}-L are known to those skilled in the art and include the use of a solvent e.g. acetone in the presence of a base, e.g. potassium carbonate.

Suitable conditions for the reduction of the primary amide to give an amine of formula (XIII) are well known and include, for example lithium aluminium hydride in THF.

Suitable reaction conditions for the condensation of (XI) and (XII) to give a pyrrole of formula (X) are known to the skilled person and include ethyl acetate/acetic acid at ambient temperature.

Suitable conditions for the deprotection of an ester (X) to give the corresponding carboxylic acid of formula (1c) are known to those skilled in the art.

Accordingly the present invention also provides a process for the preparation of a compound of formula (lc) or a derivative thereof: 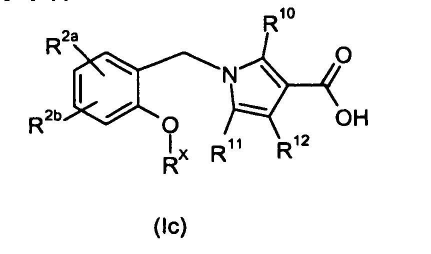 wherein:
R^{2a} and R^{2b} independently represents hydrogen, halo, optionally substituted alkyl, optionally substituted alkoxy, CN, SO₂alkyl, SR⁵, NO₂, optionally substituted aryl, CONR⁵R⁶ or optionally substituted heteroaryl;
R^{x} represents optionally substituted alkyl wherein 1 or 2 of the non-terminal carbon atoms are optionally substituted by a group independently selected from NR⁴, O and SOₙ, wherein n is 0, 1 or 2: or R^{x} may be optionally substituted CQ^{a}Q^{b}-heterocyclyl, optionally substituted CQ^{a}Q^{b}-bicyclic heterocyclyl or optionally substituted CQ^{a}Q^{b}-aryl;
R⁴ represents hydrogen or an optionally substituted alkyl;
R⁵ represents hydrogen or an optionally substituted alkyl;
R⁶ represents hydrogen or optionally substituted alkyl, optionally substituted heteroaryl, optionally substituted SO₂aryl, optionally substituted SO₂alkyl, optionally substituted SO₂heteroaryl, CN, optionally substituted CQ^{a}Q^{b}aryl, optionally substituted CQ^{a}Q^{b}heteroaryl or COR⁷;
R⁷ represents hydrogen, optionally substituted alkyl, optionally substituted heteroaryl or optionally substituted aryl;
R¹⁰ represents hydrogen, halogen, optionally substituted alkyl, optionally substituted aryl, or optionally substituted heterocyclyl;
R¹¹ represents hydrogen, halogen, optionally substituted alkyl, optionally substituted aryl, or optionally substituted heterocyclyl; and
R¹² represents hydrogen, halogen, CH₃ or CF₃;
wherein Q^{a} and Q^{b} are each independently selected from hydrogen, CH₃ and fluorine;
comprising:
reacting a compound of formula (XII): 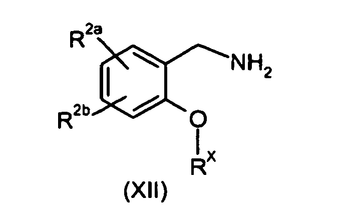
wherein R^{2a}, R^{2b}, and R^{x} are as defined above for a compound of formula (Ib);
with a compound of formula (XI): 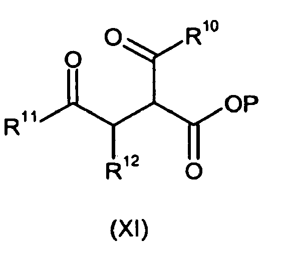 wherein R¹⁰, R¹¹, and R¹² are as defined for compounds of formula (I) and P is a protecting group;
removing the protecting group;
and, if required, forming a derivative thereof.

Compounds R^{x}-L and compounds of formula (III), (V), (VII), (IX) and t-butyl carbazate are commercially available, or may be readily prepared from commercially available intermediates by methods known to those skilled in the art.

Compounds of formula R^{x}-L wherein L is as defined above and R^{x} is as defined for compounds of formula (I) are commercially available, or may be readily prepared by known transformations of commercially available compounds.

### Compounds of formula (III):

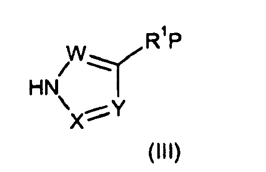

wherein W, X, Y and R¹ are as defined for compounds of formula (I) and P is an optional protecting group are commercially available, or may be prepared by conventional processes for the preparation of pyrroles, pyrazoles, triazoles and tetrazoles. The preparation of pyrroles, pyrazoles, tetrazoles and triazoles is reviewed in e.g. Comprehensive heterocyclic chemistry. The structure, reactions, synthesis and uses of heterocyclic compounds, A.R. Katritzky and C.W. Rees (Eds), vols 1-8, Pergamon Press, Oxford, 1984; Comprehensive organic chemistry II. A review of the literature 1982-1995, A.R. Katritzky, C.W. Rees, and E.F.V. Scriven (eds), vols 1-11, Pergamon Press, Oxford, 1996, and Heterocyclic Chemistry, 4th Edition, J.A. Joule and K. Mills, Blackwell Science, 2000.

### Compounds of formula (V):

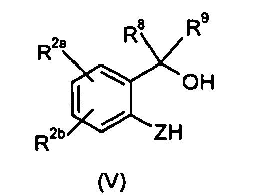

wherein Z, R^{2a}, R^{2b}, R⁸, and R⁹ are as defined for compounds of formula (I) are commercially available, or may be prepared from commercially available intermediates by conventional methods. For example, processes for the preparation of 2-(hydroxymethyl)phenols are described in W.A. Sheppard, J. Org. Chem.; 1968, 33, 3297-3306.

### Intermediates of formula (VII):

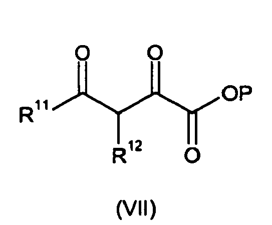

wherein R¹¹ and R¹² are as defined for compounds of formula (Ia), and P is C₁₋₄ alkyl e.g. methyl or ethyl, are commercially available or may be prepared from commercial intermediates by known processes for the preparation of 1,3-diketones e.g. J. Royals, J. Amer. Chem. Soc. 1945, 67, 1508.

### Intermediates of formula (IX):

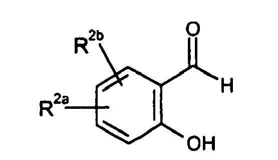

wherein R^{2a} and R^{2b} are as defined for compounds of formula (I) are commercially available, or may readily be prepared by methods known to those skilled in the art, for example from suitable commercially available starting materials using methods as described in the examples. The preparation of aldehydes is reviewed in The Chemistry of the Carbonyl Group, S. Patai (Ed), Interscience, New York, 1966, and references cited therein.

### Intermediates of formula (XI):

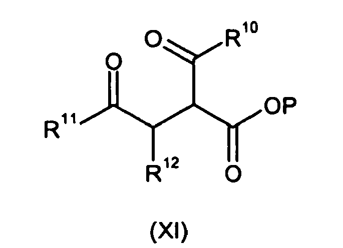

wherein R¹⁰, R¹¹ and R¹² are as defined for compounds of formula (I) are commercially available, or may readily be prepared by methods known to those skilled in the art, from suitable commercially available starting materials using methods as described in the examples.

### Intermediates of formula (XIII):

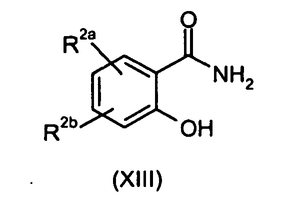

wherein R^{2a} and R^{2b} are as defined for compounds of formula (I) are commercially available, or may readily be prepared by methods known to those skilled in the art, for example from suitable commercially available starting materials. The preparation of benzamides is reviewed in The Chemistry of the Amides, Zabicky (Ed), Interscience, New York, 1970, and references cited therein.

Certain substituents in any of the reaction intermediates and compounds of formula (I) may be converted to other substituents by conventional methods known to those skilled in the art. Examples of such transformations include the reduction of a nitro group to give an amino group; alkylation and amidation of amino groups; hydrolysis of esters, alkylation of hydroxy and amino groups; and amidation and esterification of carboxylic acids. Such transformations are well known to those skilled in the art and are described in for example, Richard Larock, Comprehensive Organic Transformations, 2nd edition, Wiley-VCH, ISBN 0-471-19031-4. Fluorination of pyrazoles is described in e.g. K. Makino et al, J. Fluor. Chem, 1988, 39, 435-440. When R¹⁰ is alkyl, the R¹⁰ group may be incorporated via C-metallation and alkylation as described in, for example, Heterocyclic Chemistry, 4th Edition, J.A. Joule and K. Mills, Blackwell Science, 2000.

For example, when R^{x} is p-methoxybenzyl, cleavage of the ether to give the phenol is carried out using, for example, using acid e.g. HCl/dioxane or using sodium methanethiolate. Conversion to another R^{x} group, for example a substituted benzyl group, may be effected by reaction of the phenol with a suitable substituted benzyl bromide. The skilled person will appreciate that conversion of the protecting group P to another protecting group P may also occur under the reaction conditions used. When R^{x} is benzyl, cleavage of the ether to give the phenol may be carried out by hydrogenation according to known methods e.g. H₂Pd/C or NH₄CO₂H-Pd/C. The resulting phenol can then be converted to another group R^{x} as described above.

It will be appreciated by those skilled in the art that it may be necessary to protect certain reactive substituents during some of the above procedures. The skilled person will recognise when a protecting group is required. Standard protection and deprotection techniques, such as those described in Greene T.W. 'Protective groups in organic synthesis', New York, Wiley (1981), can be used. For example, carboxylic acid groups can be protected as esters. Deprotection of such groups is achieved using conventional procedures known in the art. It will be appreciated that protecting groups may be interconverted by conventional means.

It is to be understood that the present invention encompasses all isomers of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoismers, including mixtures thereof. The different isomeric forms may be separated or resolved one from the other by conventional methods, or any given isomer may be obtained by conventional synthetic methods or by stereospecific or asymmetric syntheses.

The compounds of the invention bind to the EP₁ receptor and are therefore considered useful in treating conditions mediated by the action of PGE₂ at EP₁ receptors.

Conditions mediated by the action of PGE₂ at EP₁ receptors include pain; fever; inflammation; immunological diseases; abnormal platelet function diseases; impotence or erectile dysfunction; bone disease; hemodynamic side effects of non-steroidal anti-inflammatory drugs; cardiovascular diseases; neurodegenerative diseases and neurodegeneration; neurodegeneration following trauma; tinnitus; dependence on a dependence-inducing agent; complications of Type I diabetes; and kidney dysfunction.

The compounds of formula (I) are considered to be useful as analgesics. They are therefore considered useful in the treatment or prevention of pain.

The compounds of formula (I) are considered useful as analgesics to treat acute pain, chronic pain, neuropathic pain, inflammatory pain, visceral pain, pain associated with cancer and fibromyalgia, pain associated with migraine, tension headache and cluster headaches, and pain associated with functional bowel disorders, non-cardiac chest pain and non-ulcer dispepsia.

The compounds of formula (I) are considered useful in the treatment of chronic articular pain (e.g. rheumatoid arthritis, osteoarthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; and dysmenorrhea. The compounds of the invention may also be considered useful in the treatment of visceral pain.

The compounds of the invention are considered to be particularly useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of formula (I) are also considered useful in the treatment of fever.

The compounds of formula (I) are also considered useful in the treatment of inflammation, for example in the treatment of skin conditions (e.g. sunburn, bums, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease, (COPD); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastrointestinal reflux disease); organ transplantation; other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, gingivitis, myocardial ischemia, pyrexia, systemic lupus erythematosus, polymyositis, tendinitis, bursitis, and Sjogren's syndrome.

The compounds of formula (I) are also considered useful in the treatment of immunological diseases such as autoimmune diseases, immunological deficiency diseases or organ transplantation. The compounds of formula (I) are also effective in increasing the latency of HIV infection.

The compounds of formula (I) are also considered useful in the treatment of diseases relating to abnormal platelet function (e.g. occlusive vascular diseases).

The compounds of formula (I) are also considered useful for the preparation of a drug with diuretic action.

The compounds of formula (I) are also considered useful in the treatment of impotence or erectile dysfunction.

The compounds of formula (I) are also considered useful in the treatment of bone disease characterised by abnormal bone metabolism or resorbtion such as osteoporosis (especially postmenopausal osteoporosis), hyper-calcemia, hyperparathyroidism, Paget's bone diseases, osteolysis, hypercalcemia of malignancy with or without bone metastases, rheumatoid arthritis, periodontitis, osteoarthritis, ostealgia, osteopenia, cancer cacchexia, calculosis, lithiasis (especially urolithiasis), solid carcinoma, gout and ankylosing spondylitis, tendinitis and bursitis.

The compounds of formula (I) are also considered useful for attenuating the hemodynamic side effects of non-steroidal anti-inflammatory drugs (NSAID's) and cyclooxygenase-2 (COX-2) inhibitors.

The compounds of formula (I) are also considered useful in the treatment of cardiovascular diseases such as hypertension or myocardiac ischemia; functional or organic venous insufficiency; varicose therapy; haemorrhoids; and shock states associated with a marked drop in arterial pressure (e.g. septic shock).

The compounds of formula (I) are also considered useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, ALS, motor neuron disease); vascular dementia (including multi-infarct dementia); as well as dementia associated with intracranial space occupying lesions; trauma; infections and related conditions (including HIV infection); metabolism; toxins; anoxia and vitamin deficiency; and mild cognitive impairment associated with ageing, particularly Age Associated Memory Impairment.

The compounds of formula (I) are also considered useful in the treatment of neuroprotection and in the treatment of neurodegeneration following trauma such as stroke, cardiac arrest, pulmonary bypass, traumatic brain injury, spinal cord injury or the like.

The compounds of formula (I) are also considered useful in the treatment of tinnitus.

The compounds of formula (I) are also considered useful in preventing or reducing dependence on, or preventing or reducing tolerance or reverse tolerance to, a dependence - inducing agent. Examples of dependence inducing agents include opioids (e.g. morphine), CNS depressants (e.g. ethanol), psychostimulants (e.g. cocaine) and nicotine.

The compounds of formula (I) are also considered useful in the treatment of complications of Type 1 diabetes (e.g. diabetic microangiopathy, diabetic retinopathy, diabetic nephropathy, macular degeneration, glaucoma), nephrotic syndrome, aplastic anaemia, uveitis, Kawasaki disease and sarcoidosis.

The compounds of formula (I) are also considered useful in the treatment of kidney dysfunction (nephritis, particularly mesangial proliferative glomerulonephritis, nephritic syndrome), liver dysfunction (hepatitis, cirrhosis), gastrointestinal dysfunction (diarrhoea) and colon cancer.

The compounds of formula (I) are also useful in the treatment of overactive bladder and urge incontenance.

It is to be understood that reference to treatment includes both treatment of established symptoms and prophylactic treatment, unless explicitly stated otherwise.

According to a further aspect of the invention, we provide a compound of formula (I) or a pharmaceutically acceptable derivative thereof for use in human or veterinary medicine.

According to another aspect of the invention, we provide a compound of formula (I) or a pharmaceutically acceptable derivative thereof for use in the treatment of a condition which is mediated by the action of PGE₂ at EP₁ receptors.

According to another aspect of the invention, we provide the use of a compound of formula (I) or a pharmaceutically acceptable derivative thereof for the manufacture of a medicament for the treatment of a condition which is mediated by the action of PGE₂ at EP₁ receptors.

According to another aspect of the invention we provide the use of a compound of formula (I) or a pharmaceutically acceptable derivative thereof for the manufacture of a medicament for the treatment or prevention of a condition such as a pain, inflammatory, immunological, bone, neurodegenerative or renal disorder.

According to another aspect of the invention we provide the use of a compound of formula (I) or a pharmaceutically acceptable derivative thereof for the manufacture of a medicament for the treatment or prevention of a condition such as inflammatory pain, neuropathic pain or visceral pain.

The compounds of formula (I) and their pharmaceutically acceptable derivatives are conveniently administered in the form of pharmaceutical compositions. Such compositions may conveniently be presented for use in conventional manner in admixture with one or more physiologically acceptable carriers or excipients.

Thus, in another aspect of the invention, we provide a pharmaceutical composition comprising a compound of formula (I) or a pharmaceutically acceptable derivative thereof adapted for use in human or veterinary medicine.

The compounds of formula (I) and their pharmaceutically acceptable derivatives may be formulated for administration in any suitable manner. They may be formulated for administration by inhalation or for oral, topical, transdermal or parenteral administration. The pharmaceutical composition may be in a form such that it can effect controlled release of the compounds of formula (I) and their pharmaceutically acceptable derivatives.

For oral administration, the pharmaceutical composition may take the form of, for example, tablets (including sub-lingual tablets), capsules, powders, solutions, syrups or suspensions prepared by conventional means with acceptable excipients.

For transdermal administration, the pharmaceutical composition may be given in the form of a transdermal patch, such as a transdermal iontophoretic patch.

For parenteral administration, the pharmaceutical composition may be given as an injection or a continuous infusion (e.g. intravenously, intravascularly or subcutaneously). The compositions may take such forms as suspensions, solutions or emulsions in oily or aqueous vehicles and may contain formulatory agents such as suspending, stabilising and/or dispersing agents. For administration by injection these may take the form of a unit dose presentation or as a multidose presentation preferably with an added preservative. Alternatively for parenteral administration the active ingredient may be in powder form for reconstitution with a suitable vehicle.

The compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

The EP₁ receptor compounds for use in the instant invention may be used in combination with other therapeutic agents, for example COX-2 inhibitors, such as celecoxib, deracoxib, rofecoxib, valdecoxib, parecoxib or COX-189; 5-lipoxygenase inhibitors; NSAID's, such as diclofenac, indomethacin, nabumetone or ibuprofen; leukotriene receptor antagonists; DMARD's such as methotrexate; adenosine A1 receptor agonists; sodium channel blockers, such as lamotrigine; NMDA receptor modulators, such as glycine receptor antagonists; gabapentin and related compounds; tricyclic antidepressants such as amitriptyline; neurone stabilising antiepileptic drugs; mono-aminergic uptake inhibitors such as venlafaxine; opioid analgesics; local anaesthetics; 5HT₁ agonists, such as triptans, for example sumatriptan, naratriptan, zolmitriptan, eletriptan, frovatriptan, almotriptan or rizatriptan; nicotinic acetyl choline (nACh) receptor modulators; glutamate receptor modulators, for example modulators of the NR2B subtype; EP₄ receptor ligands; EP₂ receptor ligands; EP₃ receptor ligands; EP₄ agonists and EP₂ agonists ; EP₄ antagonists; EP₂ antagonists and EP₃ antagonists; cannabanoid receptor ligands; bradykinin receptor ligands and vanilloid receptor ligands. When the compounds are used in combination with other therapeutic agents, the compounds may be administered either sequentially or simultaneously by any convenient route.

Additional COX-2 inhibitors are disclosed in US Patent Nos. 5,474,995 US5,633,272; US5,466,823, US6,310,099 and US6,291,523; and in WO 96/25405, WO 97/38986, WO 98/03484, WO 97/14691, WO99/12930, WO00/26216, WO00/52008, WO00/38311, WO01/58881 and WO02/18374.

The invention thus provides, in a further aspect, a combination comprising a compound of formula (I) or a pharmaceutically acceptable derivative thereof together with a further therapeutic agent or agents.

In addition to activity at the EP₁ receptor, the compounds of the present invention and pharmaceutically acceptable derivatives thereof exhibit antagonism of the TP receptor and are therefore indicated to be useful in treating conditions mediated by the action of thromboxane at the TP receptor.

In view of their antagonism of the TP receptor, the compounds of the invention and pharmaceutically acceptable derivatives thereof are indicated to be useful in the treatment of renal disorders, asthma, or gastric lesions.

Certain compounds of the invention are equipotent antagonists of the EP₁ and TP receptors.

The present invention therefore also provides a compound which is an equipotent antagonist of the TP receptor and the EP₁ receptor.

According to another aspect of the invention, we provide a compound of formula (I) or a pharmaceutically acceptable derivative thereof for use in the treatment of a condition which is mediated by the action of thromboxane at the TP receptor.

According to another aspect of the invention, we provide the use of a compound of formula (I) or a pharmaceutically acceptable derivative thereof for the manufacture of a medicament for the treatment of a condition which is mediated by the action of thromboxane at the TP receptor.

According to another aspect of the invention we provide the use of a compound of formula (I) or a pharmaceutically acceptable derivative thereof for the manufacture of a medicament for the treatment or prevention of a condition such as a renal disorder, asthma, or gastric lesions.

In certain situations it is envisaged that the administration of a compound exhibiting antagonism of TP receptors in combination with a compound exhibiting antagonism of EP₁ receptors may be advantageous.

The present invention therefore also provides a composition comprising an EP₁ antagonist or a pharmaceutically acceptable derivative thereof and a TP antagonist or a pharmaceutically acceptable derivative thereof.

According to a further aspect, we provide a combination comprising an EP₁ antagonist or a pharmaceutically acceptable derivative thereof and a TP antagonist or a pharmaceutically acceptable derivative thereof for use in the treatment of a condition which is mediated by the action of PGE₂ at EP₁ receptors.

The present invention also provides a combination comprising an EP₁ antagonist or a pharmaceutically acceptable derivative thereof and a TP antagonist or a pharmaceutically acceptable derivative thereof for use in the treatment of pain, or inflammatory, immunological, bone, neurodegenerative or renal disorders.

The present invention further provides a combination comprising an EP₁ antagonist or a pharmaceutically acceptable derivative thereof and a TP antagonist or a pharmaceutically acceptable derivative thereof for use in the treatment of inflammatory pain, neuropathic pain or visceral pain.

According to another aspect of the invention, we provide the use an EP₁ antagonist or a pharmaceutically acceptable derivative thereof in combination with a TP antagonist or a pharmaceutically acceptable derivative thereof for the manufacture of a medicament for the treatment of a condition which is mediated by the action of PGE₂ at EP₁ receptors.

According to yet another aspect of the invention we provide the use an EP₁ antagonist or a pharmaceutically acceptable derivative thereof in combination with a TP antagonist or a pharmaceutically acceptable derivative thereof for the manufacture of a medicament for the treatment of or prevention of a condition such as a pain, or an inflammatory, immunological, bone, neurodegenerative or renal disorder.

According to a further aspect of the invention we provide the use of use an EP₁ antagonist or a pharmaceutically acceptable derivative thereof in combination with a TP antagonist or a pharmaceutically acceptable derivative thereof for the manufacture of a medicament for the treatment or prevention of a condition such as inflammatory pain, neuropathic pain or visceral pain.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A proposed daily dosage of compounds of formula (I) or their pharmaceutically acceptable derivatives for the treatment of man is from 0.01 to 30 mg/kg body weight per day and more particularly 0.1 to 10 mg/kg body weight per day, which may be administered as a single or divided dose, for example one to four times per day The dose range for adult human beings is generally from 8 to 2000 mg/day, such as from 20 to 1000 mg/day, preferably 35 to 200 mg/day.

The precise amount of the compounds of formula (I) administered to a host, particularly a human patient, will be the responsibility of the attendant physician. However, the dose employed will depend on a number of factors including the age and sex of the patient, the precise condition being treated and its severity, and the route of administration.

No unacceptable toxicological effects are expected with compounds of the invention when administered in accordance with the invention.

The following non-limiting Examples illustrate the preparation of pharmacologically active compounds of the invention.

### EXAMPLES

### Abbreviations

Bn (benzyl), Bu, Pr, Me, Et (butyl, propyl, methyl ethyl), DMSO (dimethyl sulfoxide), DCM (dichloromethane), EDTA (ethylenediamine tetraacetic acid), EtOAc (ethyl acetate), EtOH (ethanol), HPLC (High pressure liquid chromatography), LCMS (Liquid chromatography/Mass spectroscopy), MDAP (Mass Directed Purification), MeCN (acetonitrile), MeOH (methanol), NMR (Nuclear Magnetic Resonance (spectrum)), Ph (phenyl), SPE (Solid Phase Extraction), THF (tetrahydrofuran), s, d, t, q, m, br (singlet, doublet, triplet, quartet, multiplet, broad.)

### LCMS

- Column: 3.3cm x 4.6mm ID, 3um ABZ+PLUS
- Flow Rate: 3ml/min
- Injection Volume: 5µl
- Temp: RT
- UV Detection Range: 215 to 330nm
- Solvents: A: 0.1% Formic Acid + 10mMolar Ammonium Acetate.
   B: 95% Acetonitrile + 0.05% Formic Acid

| Gradient: | Time | A% | B% |
|---|---|---|---|
| | 0.00 | 100 | 0 |
| | 0.70 | 100 | 0 |
| | 4.20 | 0 | 100 |
| | 5.30 | 0 | 100 |
| | 5.50 | 100 | 0 |

### Mass Directed Autopreparation

### Hardware:

Waters 600 gradient pump
Waters 2767 inject/collector
Waters Reagent Manager
Micromass ZMD mass spectrometer
Gilson Aspec - waste collector
Gilson 115 post-fraction UV detector

### Software:

Micromass Masslynx version 4.0

### Column

The column used is typically a Supelco LCABZ++ column whose dimensions are 20mm internal diameter by 100mm in length. The stationary phase particle size is 5µm.

### Solvents:

A:. Aqueous solvent = Water + 0.1 % Formic Acid
B: Organic solvent = MeCN: Water 95:5 +0.05% Formic Acid
Make up solvent = MeOH: Water 80:20 +50mMol Ammonium Acetate
Needle rinse solvent = MeOH: Water: DMSO 80:10:10

The method used depends on the analytical retention time of the compound of interest. 15-minute runtime, which comprises a 10-minute gradient followed by a 5-minute column flush and re-equilibration step.
MDP 1.5-2.2 = 0-30%B
MDP 2.0-2.8 = 5-30% B
MDP 2.5-3.0 = 15-55%B
MDP 2.8-4.0 = 30-80% B
MDP 3.8-5.5 = 50-90% B

### Flow rate:

flow rate 20ml/min.

### General Method 1

### Preparation of 4-bromo-2-(bromomethyl)phenyl phenylmethyl ether (Intermediate A)

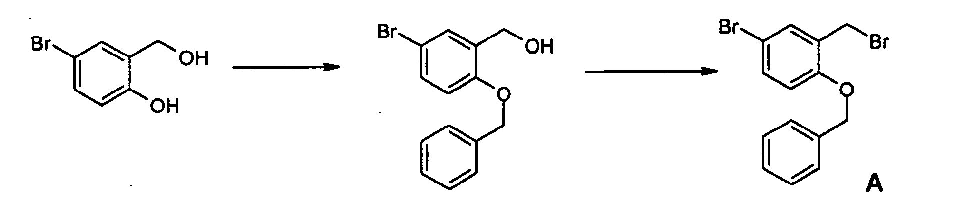

### a) {5-bromo-2-[(phenylmethyl)oxy]phenyl}methanol

4-bromo-2-(hydroxymethyl)phenol (10.15g, 50mmol) was dissolved in ethanol (100ml) and 2M sodium hydroxide (27.5ml, 55mmol). The resulting solution was stirred for 10 minutes. A solution of benzyl bromide (5.95ml, 50mmol) in ethanol (100ml) was added slowly and the resulting solution was stirred overnight at room temperature. The reaction mixture was concentrated *in vacuo,* the solution obtained diluted with water and extracted with dichloromethane. The combined organic layers were washed sequentially with a saturated solution of NaHCO₃ and water, dried (Na₂SO₄) filtered and evaporated to dryness. The residue was purified by flash chromatography using dichloromethane to yield the title compound as a colourless oil (13.8g, 94%).
¹H NMR δ: 2.19 (1 H, t), 4.71 (2H, d, J = 6.3Hz), 5.10 (2H, s), 6.82 (1 H, d, J = 8.6Hz), 7.34-7.47 (7H, m).

### b) 4-bromo-2-(bromomethyl)phenyl phenylmethyl ether (Intermediate A)

A solution of {5-bromo-2-[(phenylmethyl)oxy]phenyl}methanol (5.41g, 18.44mmol) in dichloromethane (30ml) was stirred under nitrogen and cooled to -10°C (ice/acetone). A solution of phosphorous tribromide (4.99g, 1.75ml, 18.44mmol) in dichloromethane (15ml) was added slowly at -10°C and the mixture warmed to -7°C and stirred for 15 mins. The reaction was then allowed to warm to room temperature and was stirred overnight under nitrogen. The reaction mixture was cooled (ice/water bath) and a saturated sodium hydrogen carbonate solution (15.5ml) was then added slowly and the mixture diluted with dichloromethane and water. The organic phase was separated, washed with water then dried (Na₂SO₄) and evaporated to dryness. The residue was purified by flash chromatography with diethyl ether to yield the title compound as a white solid (5.53g, 84%).
¹H NMR δ: 4.54 (2H, s), 5.15 (2H, s), 6.81 (1H, d, J = 8.8Hz), 7.33-7.48 (7H, m)

The following example was prepared using General Method 1 (b) from {5-chloro-2-[(phenylmethyl)oxy]phenyl}methanol.

### 4-chloro-2-(bromomethyl)phenyl phenylmethyl ether

t = 3.27, no ion observed.

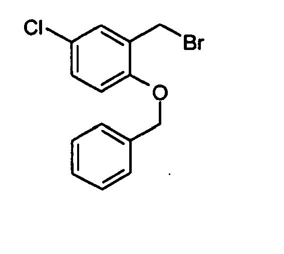

### General Method 2

### Example 1: 1-({5-bromo-2-[(phenylmethyl)oxy]phenyl}methyl)-5-methyl-1H-pyrazole-3-carboxylic acid

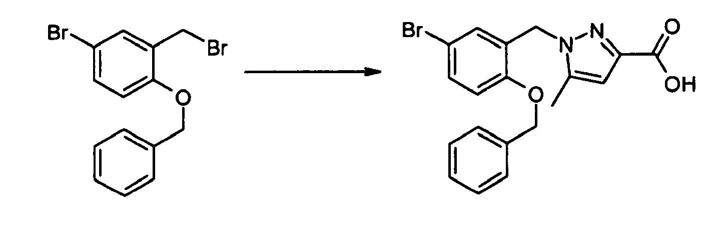

Methyl 1*H*-pyrazole-3-carboxylate (12.61 mg, 0.1 mmol) was dissolved in a 0.105M solution of potassium *tert*-butoxide in ethanol (1ml, 11.78 mg, 0.105 mmol). After stirring at room temperature for 5 mins, a 0.1 M solution of 4-bromo-2-(bromomethyl)phenyl phenylmethyl ether in ethanol (1ml, 35.6 mg, 0.1 mmol) was added and the resulting solution was stirred and heated at 60°C under nitrogen for 4hrs. After cooling the mixture was diluted with ethanol (1 ml) and a 0.5M solution of lithium hydroxide in water (1 ml, 11.97mg, 0.5mmol) was added. The mixture was stirred overnight at 40°C. After cooling 2M hydrochloric acid (0.3ml, 0.6mmol) was added and the mixture was diluted with water. Dichloromethane was added and the mixture stirred vigorously. The organic layer was separated and the solvent removed in vacuo. The residue was purified by mass directed autopurification to yield the title compound.
1-({5-bromo-2-[(phenylmethyl)oxy]phenyl}methyl)-5-methyl-1*H*-pyrazole-3-carboxylic acid: (10.7mg, 27.6%).
¹H NMR δ: 5.08 (2H, s), 5.34 (2H, s), 6.72 (1H, d, J = 2.2Hz), 6.92 (1H, d, J = 8.8Hz), 7.23 (1H, d, J = 2Hz), 7.30-7.39 (6H, m), 7.45 (1H, d, J = 2Hz).
t = 3.38, [MH+] 387, 389 [MH-] 385, 387.

### 1-({5-chloro-2-[(Phenylmethyl)oxy]phenyl}methyl)-1H-pyrazole-3-carboxylic acid ethyl ester

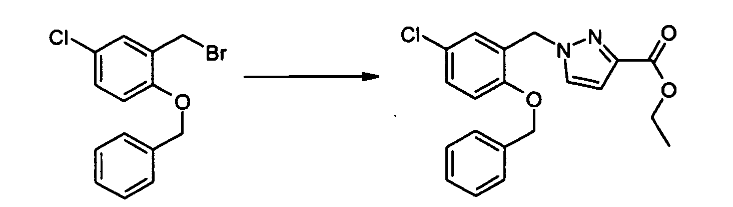

1*H*-pyrazole-3-carboxylic acid ethyl ester (13.0g, 93 mmol) was dissolved in dimethylformamide (200ml). Potassium carbonate (32g, 232 mmol) was added to the solution, followed by 4-chloro-2-(bromomethyl)phenyl phenylmethyl ether (29g, 93 mmol) and the reaction mixture stirred overnight at room temperature under argon. Water and ethyl acetate were added and the layers separated. The aqueous phase was re-extracted with ethyl acetate. The organic phases were combined and washed with water followed by brine. The extracts were dried (Na₂SO₄) and evaporated. The residue was purified by on a biotage (15-25% ethyl acetate : hexane) to yield the title compounds. 1-({5-chloro-2-[(phenylmethyl)oxy]phenyl}methyl)-1*H*-pyrazole-3-carboxylic acid ethyl ester: (13.90g, 40%).
t = 3.40, no ion observed.

### Example 2: 1-({5-chloro-2-[(phenylmethyl)oxy]phenyl}methyl)-1H-pyrazole-3-carboxylic acid

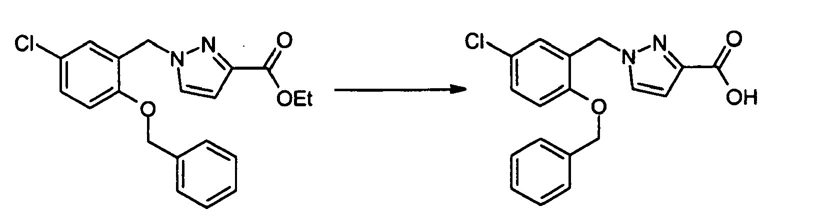

1-({5-chloro-2-[(phenylmethyl)oxy]phenyl}methyl)-1*H*-pyrazole-3-carboxylic acid ethyl ester (13.9g, 37.5mmol) was dissolved in ethanol (150ml) and 2M sodium hydroxide (45ml, 90mmol). This mixture was stirred at reflux for 4 hours. The ethanol was evaporated and the mixture diluted with ethyl acetate and water. This was acidified with 2M hydrochloric acid, and the phases separated. The aqueous phase was re-extracted with ethyl acetate, the organic layers combined, dried (Na₂SO₄) and evaporated to dryness, to give the title compound as a white solid (12.09g, 94%).
t = 2.98, [MH-] 341.

### General Method 3

### Preparation of ethyl 1-[(5-bromo-2-hydroxyphenyl)methyl]-5-methyl-1H-pyrazole-3-carboxylate (Intermediate B)

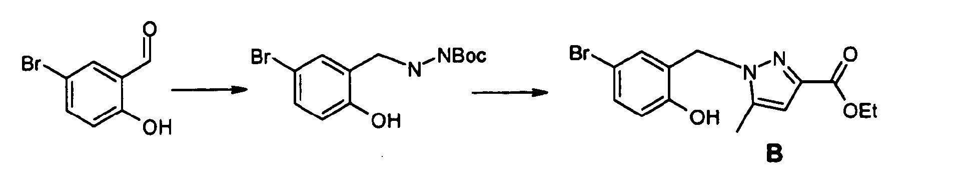

### a) 1,1-dimethylethyl 2-[(5-bromo-2-hydroxyphenyl)methyl]hydrazinecarboxylate

5-bromo-2-hydroxybenzaldehyde (4.02g, 20mmol) was dissolved in dichloromethane (100ml). *Tert*-butyl carbazate (2.64g, 20mmol) and acetic acid (1.14ml, 1.2g, 20mmol) were added and the mixture was stirred under nitrogen for 30mins. Sodium triacetoxyborohydride (12.72g, 60mmol) was added portionwise and the resulting suspension was then stirred overnight under nitrogen. 2M hydrochloric acid (30ml, 60mmol) was added and the resulting solution was diluted with dichloromethane and water. The organic phase was separated, washed sequentially with brine and water then dried (Na₂SO₄) and evaporated to dryness to give the title compound as a white solid (6.01g, 94.7%)
¹H NMR δ: 1.48 (9H, s), 4.13 (2H, s), 4.40 (1H, br s), 6.15 (1 H, br s), 6.78 (1H, d, J = 8.8Hz), 7.16 (1H, d, J = 2.26Hz), 7.29-7.32 (1 H, m), 9.28 (1 H, br s).
t = 3.11, [MH+] 317, 319 [MH-] 315, 317.

### b) Ethyl 1-[(5-bromo-2-hydroxyphenyl)methyl]-5-methyl-1H-pyrazole-3-carboxylate (Intermediate B)

Trifluoroacetic acid (20ml) was added to 1,1-dimethylethyl 2-[(5-bromo-2-hydroxyphenyl)methyl]hydrazinecarboxylate (3.2g, 10mmol) in dichloromethane (40ml) and the reaction mixture stirred overnight at room temperature under nitrogen. The solvent was removed in vacuo and the residue obtained redissolved in acetic acid (20ml). The resulting solution was added dropwise to a solution of ethyl 2,4-dioxopentanoate (1.40ml, 1.58g, 10mmol) in acetic acid (10ml) and the reaction mixture was heated at reflux under nitrogen for 1 h. The title compound crystallized upon cooling, was filtered, washed with acetic acid and dried under vacuo to give the title compound as white crystals (1.85g, 54.7%)
¹H NMR δ: 1.39 (3H, t, J = 7.15 Hz), 2.41 (3H, s), 4.34-4.40 (2H, q), 5.18 (2H, s), 6.58 (1H, s), 6.88 (1 H, d, J = 8.5Hz), 7.24 (1H, d, J = 2.2Hz), 7.33 (1H, m), 9.56 (1H, br s).
t = 3.17, [MH+] 339, 341 [MH-] 337, 339.

### General Method 4

### Example 3: 1-[(5-Bromo-2-{[(2,4-difluorophenyl)methyl]oxylphenyl)methyl]-5-methyl-1H-Pyrazole-3-carboxylic acid

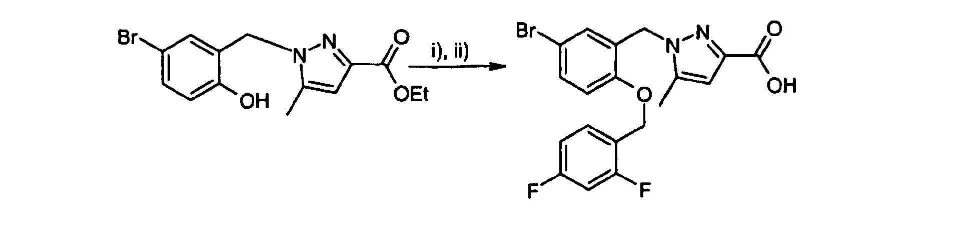

Ethyl 1-[(5-bromo-2-hydroxyphenyl)methyl]-5-methyl-1*H*-pyrazole-3-carboxylate (16.95mg, 0.05mmol) was dissolved in ethanol (0.5ml) and 2M sodium hydroxide (0.0275ml, 0.055mmol) and stirred at room temperature for 5 mins. 1-(bromomethyl)-2,4-difluorobenzene (1 0.35mg, 0.05mmol) in ethanol (0.5ml) was added and the reaction mixture heated under nitrogen at 70°C overnight. After cooling the mixture was diluted with ethanol (0.5 ml) and a 0.5M solution of lithium hydroxide in water (0.5 ml, 5.99mg, 0.25mmol) was added. The mixture was stirred at 40°C for 3 h. After cooling 2M hydrochloric acid (0.15ml, 0.3mmol) and the mixture was diluted with water. Dichloromethane was added and the mixture stirred vigorously. The organic layer was separated and the solvent removed *in vacuo.* The residue was purified by mass directed autopurification to yield the title compound (18.4mg, 84.2%).
¹H NMR δ: 2.13 (3H, s), 5.10 (2H, s), 5.27 (2H, s), 6.55 (1H, s), 6.85 (1 H, d, J = 2Hz), 6.89-6.97 (3H, m), 7.36-7.46 (2H, m).
t = 3.48, [MH+] 437, 439 [MH-] 435, 437.

### Ethyl 1-({5-chloro-2-[(2-methylpropyl)oxylphenyl)methyl)-5-methyl-1H-pyrazole-3-carboxylate

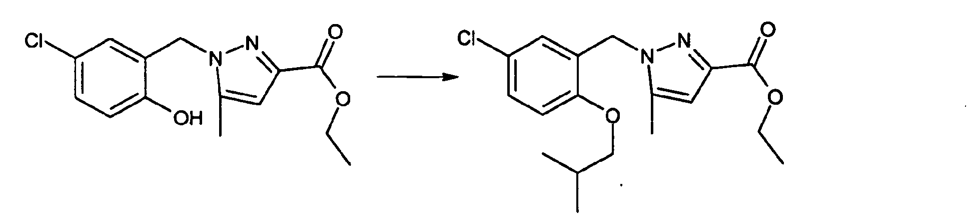

A mixture of ethyl 1-[(5-chloro-2-hydroxyphenyl)methyl]-5-methyl-1*H*-pyrazole-3-carboxylate (25.82g, 0.088mol), potassium carbonate (0.176mol, 24.3g), isobutyl bromide (0.132mol, 14.2ml) was stirred in dimethylformamide (175ml) at 116°C for 16 hours. After this time further isobutyl bromide (0-044mol, 4.7ml) was added and the reaction continued for 2 hours. This was evaporated to a solid and then partitioned between ethyl acetate (500ml) and water (200ml). The aqueous was run off and the organic washed twice with water (100ml) and once with brine (50ml) before being evaporated to a solid which was flash chromatographed with hexane/ethyl acetate (4/1) to give the title compound (29.12g) LC/MS [M+H] 351 and 353, Rt = 3.47min

### Example 4: Sodium 1-({5-chloro-2-[(2-methylpropyl)oxy]phenyl}methyl)-5-methyl-1H-pyrazole-3-carboxylate

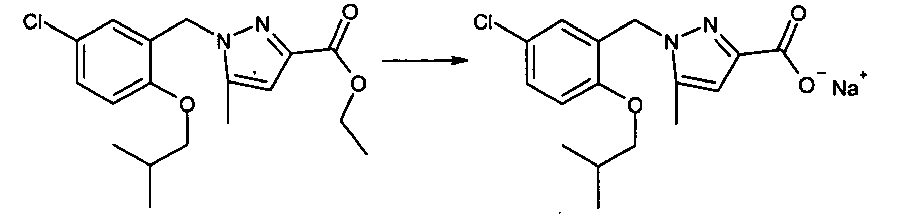

Ethyl 1-({5-chloro-2-[(2-methylpropyl)oxy]phenyl}methyl)-5-methyl-1H-pyrazole-3-carboxylate (0.083mol, 29.12g) was stirred in ethanol (330ml) and 2N sodium hydroxide (100ml) at 95°C for 1½ hours. This was cooled to room temperature and evaporated to a solid. This was partitioned between EtOAc (500ml) and water (200ml). Some of the ethyl acetate layer (5ml) was taken, washed with brine (2ml) and dried over magnesium sulphate and evaporated to give the title compound (0.225g). LC/MS [M+Na] 345 and 347, Rt = 2.94min

Regioisomers: Elucidation of isolated structures where regioisomers can be formed (general methods 2 and 3) was determined by using either NMBC (heteronuclear multiple bond correlation); nOe (nuclear Overhauser effect) NMR techniques.

The following Examples were prepared from either Intermediate A or Intermediate B and Methods 2 or 4, and other appropriate starting materials.

| | | | |
|---|---|---|---|
| 5 | 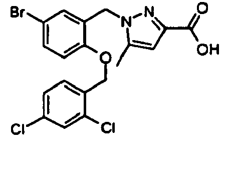 | Name | 1-{5-bromo-2-[(2,4-dichlorobenzyl)oxy]benzyl}-5-methyl-1H-pyrazole-3-carboxylic acid |
| | | NMR | ¹H NMR δ: 2.15 (3H, s), 5.13 (2H, s), 5.31 (2H, s), 6.57 (1 H, s), 6.80 (1 H, d, J = 2.5Hz), 6.86 (1 H, d, J = 8.8Hz), 7.28 (1H, m), 7.35 (1H, m), 7.39 (1H, d, J = 8.3Hz), 7.43 (1 H, d, J = 2Hz) |
| | | LCMS | t = 3.84, [MH+] 467, 469, 471 [MH-] 469, 471, 473 |
| | | Method | B and Method 4 |
| 6 | 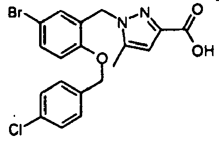 | Name | 1-{5-bromo-2-[(4-chlorobenzyl)oxy]benzyl}-5-methyl-1H-pyrazole-3-carboxylic acid |
| | | NMR | ¹H NMR δ: 2.16 (3H, s), 5.10 (2H, s), 5.32 (2H, s), 6.57 (1H, s), 6.91 (1H, d, J = 2.2Hz), 6.99 (1H, d, J = 8.8Hz), 7.38-7.40 (5H, m) |
| | | LCMS | t = 3.60, [MH+] 435, 437 [MH-] 433, 435 |
| | | Method | B and Method 4 |
| 7 | 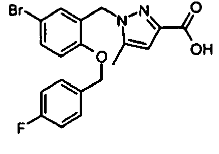 | Name | 1-{5-bromo-2-[(4-fluorobenzyl)oxy]benzyl}-5-methyl-1 H-pyrazole-3-carboxylic acid |
| | | NMR | ¹H NMR δ: 2.14 (3H, s), 5.09 (2H, s), 5.30 (2H, s), 6.57 (1H, s), 6.92 (1 H, d, J = 2Hz), 7.00 (1H, d, J = 8.8Hz), 7.10 (2H, t, J = 8.8Hz), 7.38-7.44 (3H, m) |
| | | LCMS | t = 3.44, [MH+] 419, 421 [MH-] 417, 419 |
| | | Method | B and Method 4 |
| 8 | 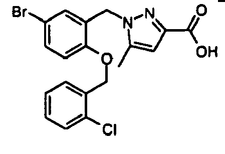 | Name | 1-{5-bromo-2-[(2-chlorobenzyl)oxy]benzyl}-5-methyl-1 H-pyrazole-3-carboxylic acid |
| | | NMR | ¹H NMR δ: 2.13 (3H, s), 5.17 (2H, s), 5.32 (2H, s), 6.56 (1H, s), 6.80 (1H, d, J = 2.3Hz), 6.88 (1H, d, J = 8.8Hz), 7.25-7.43 (5H, m) |
| | | LCMS | t = 3.66, [MH+] 435, 437 [MH-] 433, 435 |
| | | Method | B and Generic Method 4 |
| 9 | 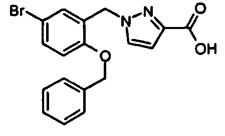 | Name | 1-[2-(benzyloxy)-5-bromobenzyl]-1H-pyrazole-3-carboxylic acid |
| | | NMR | ¹H NMR δ: 5.08 (2H, s), 5.34 (2H, s), 6.72 (1 H, d, J = 2.2Hz), 6.92 (1H, d, J = 8.8Hz), 7.23 (1 H, d, J = 2Hz), 7.30-7.39 (6H, m), 7.45 (1 H, d, J = 2Hz) |
| | | LCMS | t = 3.38, [MH+] 487, 489 [MH-] 485, 487 |
| | | Method | A and Method 2 |
| 10- | 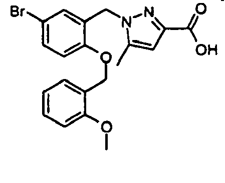 | Name | 1-{5-bromo-2-[(2-methoxybenzyl)oxy]benzyl}-5-methyl-1 H-pyrazole-3-carboxylic acid |
| | | NMR | ¹H NMR δ: 2.12 (3H, s), 3.83 (3H, s), 5.10 (2H, s), 5.28 (2H, s), 6.54 (1 H, s), 6.85 (1H, d), 6.91-6.96 (3H, m), 7.28-7.35 (3H, m) |
| | | LCMS | t = 3.52, [MH+] 431, 433 [MH-] 429, 431 |
| | | Method | B and Method 4 |
| 11 | 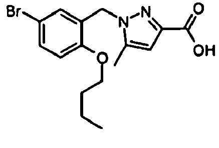 | Name | 1-(5-bromo-2-butoxybenzyl)-5-methyl-1H-pyrazole-3-carboxylic acid |
| | | NMR | ¹H NMR δ: 0.96 (3H, t, J = 7.4Hz), 1.43-1.52 (2H, m), 1.73-1.80 (2H, m), 2.21 (3H, s), 3.99 (2H, t, J = 6.4Hz), 5.28 (2H, s), 6.58 (1H, s), 6.70 (1H, d, J = 2.2Hz), 6.78 (1H, d, J = 8.8Hz), 7.29-7.32 (1H, m) |
| | | LCMS | t = 3.55, [MH+] 367, 369 [MH-] 365, 367 |
| | | Method | B and Method 4 |
| 12 | 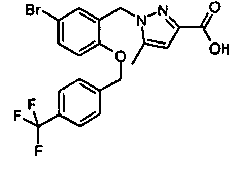 | Name | 1-(5-bromo-2-{[4-(trifluoromethyl)benzyl]oxy}benzyl)-5-methyl-1H-pyrazole-3-carboxylic acid |
| | | NMR | ¹H NMR δ: 2.18 (3H, s), 5.23 (2H, s), 5.36 (2H, s), 6.58 (1 H, s), 6.91 (1H, d, J = 2.2Hz), 7.00 (1H, d, J = 8.8Hz), 7.38-7.41 (1H, m), 7.59 (2H, d, J = 8.3Hz), 7.68 (2H, d, J = 8.3Hz) |
| | | LCMS | t = 3.61, [MH+] 469, 471 [MH-] 467, 469 |
| | | Method | B and Method 4 |
| 13 | 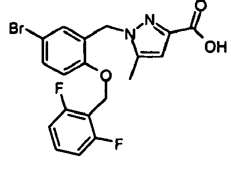 | Name | 1-{5-bromo-2-[(2,6-difluorobenzyl)oxy]benzyl}-5-methyl-1H-pyrazole-3-carboxylic acid |
| | | NMR | ¹H NMR δ: 2.08 (3H, s), 5.18 (2H, s), 5.21 (2H, s), 6.50 (1 H, s), 6.94 (1H, d, J = 2Hz), 7.04 (2H, t, J = 8Hz), 7.14 (1 H, d, J = 8.8Hz), 7.43-7.50 (2H, m) |
| | | LCMS | t = 3.43, [MH+] 437, 439 [MH-] 435, 437 |
| | | Method | B and Method 4 |
| 14 | 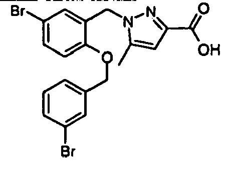 | Name | 1-{5-bromo-2-[(3-bromobenzyl}oxy]benzyl}-5-methyl-1 H-pyrazole-3-carboxylic acid |
| | | NMR | ¹H NMR δ: 2.16 (3H, s), 5.08 (2H, s), 5.31 (2H, s), 6.58 (1 H, s), 6.85 (1 H, d, J = 2.1 Hz), 6.89 (1H, d, J = 8.8Hz), 7.24-7.50 (5H, m) |
| | | LCMS | t = 3.64, [MH+] 479, 481, 483 [MH-] 477, 479, 481 |
| | | Method | B and Method 4 |
| 15 | 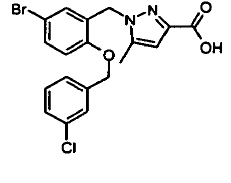 | Name | 1-{5-bromo-2-[(3-chlorobenzyl)oxy]benzyl}-5-methyl-1 H-pyrazole-3-carboxycylic acid |
| | | NMR | ¹H NMR δ: 2.16 (3H, s), 5.08 (2H, s), 5.31 (2H, s), 6.58 (1H, s), 6.85 (1H, d, J = 2Hz), 6.89 (1H, d, J = 8.8Hz), 7.27-7.36 (5H, m) |
| | | LCMS | t = 3.59, [MH+] 435, 437 [MH-] 433, 435 |
| | | Method | B and Method 4 |
| 16 | 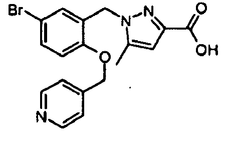 | Name | 1-[5-bromo-2-(pyridin-4-ylmethoxy)benzyl]-5-methyl-1H-pyrazole-3-carboxylic acid |
| | | NMR | ¹H NMR δ: 2.23 (3H, s), 5.22 (2H, s), 5.40 (2H, s), 6.61 (1H, s), 6.87-6.89 (2H, m), 7.35-7.38 (1H, m), 7.51 (2H, d, J = 5.27Hz), 8.53 (2H, d, J = 4.52Hz) |
| | | LCMS | t = 2.58, [MH+] 402, 404 [MH-] 400, 402 |
| | | Method | B and Method 4 |
| 17 | 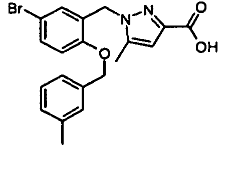 | Name | 1-{5-bromo-2-[(3-methylbenzyl)oxy]benzyl}-5-methyl-1 H-pyrazole-3-carboxylic acid |
| | | NMR | ¹H NMR δ: 2.12 (3H, s), 2.33 (3H, s), 5.03 (2H, s), 5.29 (2H, s), 6.56 (1 H, s), 6.80 (1 H, d, J = 2Hz), 6.85 (1 H, d, J = 8.8Hz), 7.11-7.25 (4H, m), 7.30-7.33 (1H, m) |
| | | LCMS | t = 3.58, [MH+] 415, 417 [MH-] 413, 415 |
| | | Method | B and Method 4 |
| 18 | 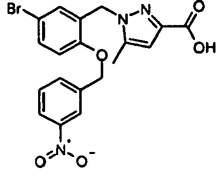 | Name | 1-{5-bromo-2-[(3-nitrobenzyl)oxy]benzyl}-5-methyl-1 H-pyrazole-3-carboxylic acid |
| | | NMR | |
| | | LCMS | t = 3.39, [MH+] 446, 448 [MH-] 444, 446 |
| | | Method | B and Method 4 |
| 19 | 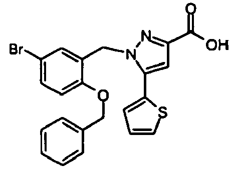 | Name | 1-[2-(benzyloxy)-5-bromobenzyl]-5-thien-2-yl-1H-pyrazole-3-carboxylic acid |
| | | NMR | ¹H NMR δ: 5.06 (2H, s), 5.50 (2H, s), 6.74 (1 H, d, J = 1.8Hz), 6.88 (1 H, d, J = 8.8Hz), 6.95 (1H, s), 7.00-7.02 (2H, m), 7.27-7.34 (6H, m), 7.43 (1H, d, J = 5.0Hz) |
| | | LCMS | t = 3.83, [MH+] 469, 471 [MH-] 467, 469 |
| | | Method | A and Method 2 |
| 20 | 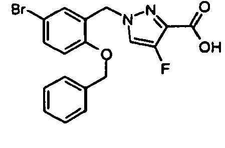 | Name | 1-[2-(benzyloxy)-5-bromobenzyl]-4-fluoro-1H-pyrazole-3-carboxylic acid |
| | | NMR | ¹H NMR δ: 5.07 (2H, s), 5.23 (2H, s), 6.90 (1H, d, J = 8.8Hz), 7.29-7.40 (8H, m) |
| | | LCMS | t = 3.48, [MH+] 405, 407 [MH-] 403, 405 |
| | | Method | A and Method 2 |

The following intermediates were prepared from the appropriate starting materials according to Method 3.

| | | | |
|---|---|---|---|
| C | 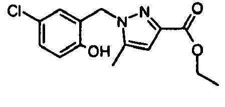 | Name | ethyl 1-[(5-chloro-2-hydroxyphenyl)methyl]-5-methyl-1 H-pyrazole-3-carboxylate |
| | | NMR | ¹H NMR δ: 1.26 (3H, t, J=6.9Hz), 2.27 (3H, s), 4.24 (2H, q, J=6.9Hz), 5.23 (2H, s), 6.58 (1 H, s), 6.64 (1H, s), 6.86 (1 H, d, J=8.5Hz), 7.17 (1 H, d, J=8.5Hz), 10.18 (1H, s) |
| | | LCMS | t=3.10, [MH+] 295, 297, [MH-] 295, 293 |
| | | Method | Method 3 |
| D | 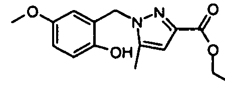 | Name | ethyl 1-{[2-hydroxy-5-(methyloxy)phenyl]methyl}-5-methyl-1 H-pyrazole-3-carboxylate |
| | | NMR | ¹H NMR δ:1.39 (3H, t, J=7.2Hz), 2.40 (3H, s), 3.75 (3H, s), 4.37 (2H, q, J=6.9Hz), 5.21 (2H, s), 6.57 (1 H, s), 6.70 (1 H, d, *J*=3.0Hz), 6.81 (1H, dd, J=3.0 and 8.9Hz), 7.93 (1 H, d, J=8.9Hz), 8.65-8.78 (1 H, br s) |
| | | LCMS | t=2.83, [MH+] 291, [MH-] 289 |
| | | Method | Method 3 |
| E | 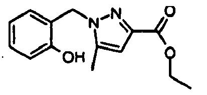 | Name | ethyl 1-[(2-hydroxyphenyl)methyl]-5-methyl-1*H*-pyrazole-3-carboxylate |
| | | NMR | ¹H NMR δ: 1.39 (3H, t, *J*=7.2Hz), 2.40 (3H, s), 4.37 (2H, q, *J*=7.2Hz), 5.25 (2H, s), 6.57 (1H, s), 6.85 - 6.89 (1 H, m), 6.99 (1 H, dd, J=7.3 and 1.0Hz), 7.14 (1 H, dd, J=7.5 and 1.5Hz), 7.24 (1H, dd, J=1.8 and 8.0Hz), 9.22 (1 H, s) |
| | | LCMS | t=2.85, [MH+] 261 |
| | | Method | Method 3 |
| F | 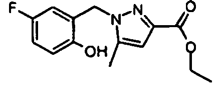 | Name | ethyl 1-[(5-fluoro-2-hydroxyphenyl)methyl]-5-methyl-1H-pyrazole-3-carboxylate |
| | | NMR | ¹H NMR δ:1.39 (3H, t, J=7.2Hz), 2.40 (3H, s), 4.37 (2H, q, J=7.2Hz), 5.20 (2H, s), 6.59 (1 H, s), 6.82 (dd, 1 H, J=2.0 and 7.5Hz), 6.93 (2H, m) |
| | | LCMS | t=2.92, [MH+] 279, 280 |
| | | Method | Method 3 |

The following Examples were prepared from either Intermediate C, D, E or F according to Method 4.

| | | | |
|---|---|---|---|
| 21 | 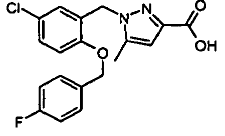 | Name | 1-[(5-chloro-2-{[(4-fluorophenyl)methyl]oxy)phenyl)methyl]-5-methyl-1 H-pyrazole-3-carboxylic acid |
| | | NMR | ¹H NMR δ: 2.16 (3H, s), 5.04 (2H, s), 5.33 (2H, s), 6.67 (1H, s), 6.71 (1H, d, J= 2.5Hz), 6.87 (1H, d, J=8.8Hz), 7.10 (2H, t, J= 8.8Hz), 7.22 (1 H, dd, J=8.8 and 2.5Hz), 7.36 (2H, dd, J=5.3 and 3.0Hz) |
| | | LCMS | t=3.37, [MH+] 375, 377, [MH-] 373, 375 |
| | | Method | C and Method 4 |
| 22 | 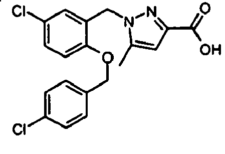 | Name | 1-[(5-chloro-2-{[(4-chlorophenyl)methyl]oxy}phenyl)methyl]-5-methyl-1*H*-pyrazole-3-carboxylic acid |
| | | NMR | ¹H NMR δ: 2.15 (3H, s), 5.10 (2H, s), 5.31 (2H, s), 6.57 (1 H, s), 6.76 (1H, d, J=2.5Hz), 7.03 (1H, d, J=8.8Hz), 7.24 (1 H, dd, J=8.8 and 2.5Hz), 7.35 -7.40 (4H, m) |
| | | LCMS | t=3.51, [MH+] 391, 393, [MH-] 389, 391 |
| | | Method | C and Method 4 |
| 23 | 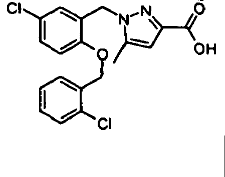 | Name | 1-[(5-chloro-2-{[(2-chlorophenyl)methyl]oxy}phenyl)methyl]-5-methyl-1H-pyrazole-3-carboxylic acid |
| | | NMR | ¹H NMR δ: 2.19 (3H, s), 5.19 (2H, s), 5.40 (2H,s), 6.67 (1 H, s), 6.79 (1H, d, J=2.3Hz), 6.89 (1 H, d, J=8.8Hz), 7.22 (1 H, dd, J=8.8 and 2.3Hz), 7.30 -7.32 (2H, m), 7.43 - 7.45 (2H, m) |
| | | LCMS | t=3.53, [MH⁺] 391, 393, [MH-] 389, 391 |
| | | Method | C and Method 4 |
| 24 | 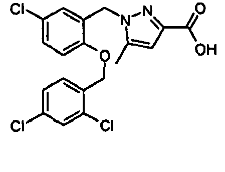 | Name | 1-[(5-chloro-2-{[(2,4-dichlorophenyl)methyl]oxy}phenyl)methyl]-5-methyl-1 *H*-pyrazole-3-carboxylic acid |
| | | NMR | ¹H NMR δ: 2.13 (3H, s), 5.20 (2H, s), 5.27 (2H, s), 6.48 (1 H,s), 6.85 (1 H, d, J=2.5Hz), 7.16 (1 H, d, J=8.8Hz), 7.38 (1H, dd, J=8.8 and 2.5Hz), 7.45 (1H, dd, J=8.3 and 2.0Hz), 7.62 (1 H, d, J=8.3Hz), 7.71 (1 H, d, J=2.3Hz) |
| | | LCMS | t=3.75, [MH+] 425, 427, 429, [MH-] 423, 425, 427 |
| | | Method | C and Method 4 |
| 25 | 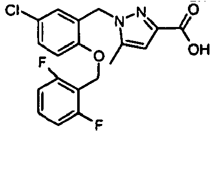 | Name | 1-[(5-chloro-2-{[(2,6-difluorophenyl)methyl]oxy)phenyl)methyl]-5-methyl-1*H*-pyrazole-3-carboxylic acid |
| | | NMR | ¹H NMR δ: 2.13 (3H, s), 5.17 (2H, s), 5.27 (2H, s), 6.63 (1 H,s), 6.73 (1 H, d, *J=*2.5Hz), 6.95 - 7.02 (3H, m), 7.25 (1 H, d, *J*=2.5Hz), 7.34 - 7.41 (1H, m) |
| | | LCMS | t=3.36, [MH+] 393,395, [MH-] 391, 393 |
| | | Method | C and Method 4 |
| 26 | 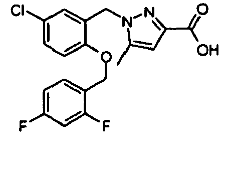 | Name | 1-[(5-chloro-2-{[(2,4-difluorophenyl)methyl]oxy}phenyl)methyl]-5-methyl-1 H-pyrazole-3-carboxylic acid |
| | | NMR | ¹H NMR δ: 2.17 (3H, s), 5.09 (2H, s), 5.33 (2H, s), 6.66 (1 H,s), 6.71 (1 H, d, J=2.5Hz), 6.86 - 6.94 (3H, m), 7.24 (1H, dd, *J*=8.8 and 2.5Hz), 7.36 -7.42 (1 H, m) |
| | | LCMS | t=3.39, [MH⁺] 393,395, [MH-] 391, 393 |
| | | Method | C and Method 4 |
| 27 | 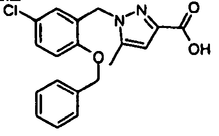 | Name | 1-({5-chloro-2-[(phenylmethyl)oxy]phenyl}methyl)-5-methyl-1 *H*-pyrazole-3-carboxylic acid |
| | | NMR | ¹H NMR δ: 2.16 (3H, s), 5.09 (2H, s), 5.36 (2H, s), 6.67 (1 H,s), 6.71 (1H, d, *J*=2.5Hz), 6.89 (1H, d, *J*=8.8Hz), 7.21 (1 H, dd, *J*=8.8 and 2.5Hz), 7.36 -7.43 (5H, m) |
| | | LCMS | t=3.35, [MH+] 357, 359, [MH-], 355, 357 |
| | | Method | C and Method 4 |
| 28 | 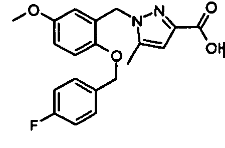 | Name | 1-{[2-{[(4-fluorophenyl)methyl]oxy}-5-(methyloxy)phenyl]methyl}-5-methyl-1 *H*-pyrazole-3-carboxylic acid |
| | | NMR | ¹H NMR δ: 2.15 (3H, s), 3.68 (3H, s), 5.01 (2H, s), 5.36 (2H, s), 6.34 (1 H, d, J=2.8Hz), 6.65 (1 H, s), 6.77 (1 H, dd, J=8.8 and 3.0Hz), 6.87 (1H, d, J= 8.8Hz), 7.06-7.11 (2H, m), 7.35 - 7.39 (2H, m) |
| | | LCMS | t=3.20, [MH+] 371, [MH-] 369 |
| | | Method | D and Method 4 |
| 29 | 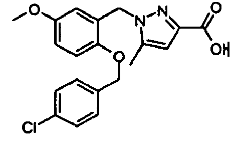 | Name | 1-{[2-{[(4-chlorophenyl)methyl]oxy}-5-(methyloxy)phenyl]methyl}-5-methyl-1*H*-pyrazole-3-carboxylic acid |
| | | NMR | ¹H NMR δ: 2.17 (3H, s), 3.68 (3H, s), 5.02 (2H, s), 5.37 (2H, s), 6.33 (1 H, d, *J*=3.0Hz), 6.66 (1 H, s), 6.76 (1 H, dd, J=8.8 and 3.0Hz), 6.85 (1H, d, J= 9.0Hz), 7.32 - 7.38 (4H, m) |
| | | LCMS | t=3.36, [MH+] 387, 389, [MH-] 385, 387 |
| | | Method | D and Method 4 |
| 30 | 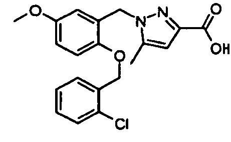 | Name | 1-{[2-{[(2-chlorophenyl)methyl]oxy}-5-(methyloxy)phenyl]methyl}-5-methyl-1*H*-pyrazole-3-carboxylic acid |
| | | NMR | ¹H NMR δ: 2.18 (3H, s), 3.68 (3H, s), 5.16 (2H, s), 5.42 (2H, s), 6.33 (1 H, d, *J*=3.0Hz), 6.65 (1H, s), 6.78 (1H, dd, J=8.8 and 3.0Hz), 6.89 (1H, d, J= 9.0Hz), 7.29 - 7.31 (2H, m), 7.42 - 7.44 (1 H, m), 7.47 - 7.49 (1H, m) |
| | | LCMS | t=3.36, [MH+] 387, 389, [MH-] 385, 387 |
| | | Method | D and Method 4 |
| 31 | 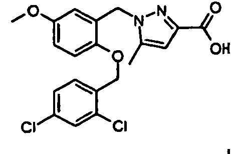 | Name | 1-{[2-{[(2,4-dichlorophenyl)methyl]oxy}-5-(methyloxy)phenyl]methyl}-5-methyl-1 H-pyrazole-3-carboxylic acid |
| | | NMR | ¹H NMR δ: 2.13 (3H, s), 3.64 (3H, s), 5.12 (2H, s), 5.27 (2H, s), 6.33 (1H, d, *J*=3.0Hz), 6.46 (1 H, s), 6.86 (1H, dd, J=9.0 and 3.3Hz), 7.04 (1H, d, J= 8.8Hz), 7.44 (1H, dd, *J*=8.3 and 2.0Hz), 7.63 (1H, d, *J*=8.3), 7.68 (1H, d, *J*=2.0Hz) |
| | | LCMS | t=3.57, [MH+] 421, 423, [MH-] 419, 421 |
| | | Method | D and Method 4 |
| 32 | 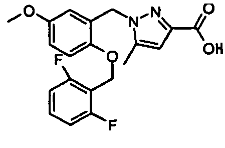 | Name | 1-{[2-{[(2,6-difluorophenyl)methyl]oxy}-5-(methyloxy)phenyl]methyl}-5-methyl-1*H*-pyrazole-3-carboxylic acid |
| | | NMR | ¹H NMR δ: 2.12 (3H, s), 3.68 (3H, s), 5.13 (2H, s), 5.30 (2H, s), 6.34 (1 H, d, J=2.8Hz), 6.61 (1H, s), 6.80 (1H, dd, J=8.8 and 3.0Hz), 6.93 - 7.03 (3H, m), 7.32 - 7.39 (1 H, m) |
| | | LCMS | t=3.20, [MH+] 389, [MH-] 387 |
| | | Method | 12 and Method 4 |
| 33 | 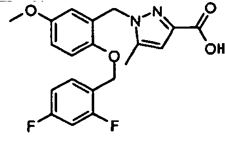 | Name | 1-{[2-{[(2,4-difluorophenyl)methyl]oxy}-5-(methyloxy)phenyl]methyl}-5-methyl-1 H-pyrazole-3-carboxylic acid |
| | | NMR | ¹H NMR 6: 2.10 (3H, s), 3.64 (3H, s), 5.08 (2H, s), 5.20 (2H, s), 6.33 (1H, d, J=3.0Hz), 6.46 (1 H, s), 6.85 (1 H, dd, J=9.0 and 3.3Hz), 7.05 - 7.09 (2H, m), 7.21 - 7.27 (1H, m), 7.56 - 7.62 (1H, m) |
| | | LCMS | t=3.23, [MH+] 389, [MH-] 387 |
| | | Method | D and Method 4 |
| 34 | 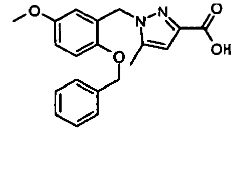 | Name | 5-methyl-1-({5-(methyloxy)-2-[(phenylmethyl)oxy]phenyl}methyl)-1H-pyrazole-3-carboxylic acid |
| | | NMR | ¹H NMR δ: 2.14 (3H, s), 3.68 (3H, s), 5.06 (2H, s), 5.38 (2H, s), 6.34 (1H, d, J=2.8Hz), 6.65 (1 H, s), 6.77 (1 H, dd, J=9.0 and 3.0Hz), 6.89 (1 H, d, J=9.0Hz), 7.35 - 7.41 (5H, m) |
| | | LCMS | t=3.18, [MH+] 353, [MH-] 351 |
| | | Method | D and Method 4 |
| 35 | 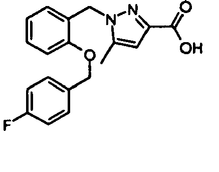 | Name | 1-[(2-{[(4-fluorophenyl)methyl]oxy}phenyl)methyl]-5-methyl-1 H-pyrazole-3-carboxylic acid |
| | | NMR | ¹H NMR δ: 2.15 (3H, s), 5.15 (2H, s), 5.28 (2H, s), 6.48 (1 H, s), 6.76 (1H, d, *J*=6.3Hz), 6.89 - 6.92 (1 H, m), 7.11 (1 H, d, *J*=8.0Hz), 7.18 - 7.23 (1 H, m), 7.25 - 7.29 (1 H, m), 7.48 - 7.52 (1 H, m) |
| | | LCMS | t=3.21, [MH+] 341, [MH-] 339 |
| | | Method | E and Method 4 |
| 36 | 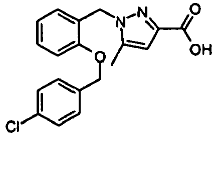 | Name | 1-[(2-{[(4-chlorophenyl)methyl]oxy}phenyl)methyl]-5-methyl-1 *H*-pyrazole-3-carboxylic acid |
| | | NMR | ¹H NMR δ: 2.17 (3H, s), 5.08 (2H, s), 5.39 (2H, s), 6.66 (1 H, s), 6.77 (1 H, d, J=7.5Hz), 6.90 - 6.93 (2H, m), 7.24 - 7.26 (2H, m), 7.33 - 7.39 (4H, m) |
| | | LCMS | t=3.38, [MH+] 357, 359, [MH-] 355, 357 |
| | | Method | E and Method 4 |
| 37 | 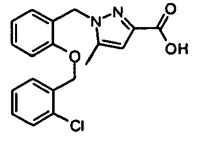 | Name | 1-[(2-{[(2-chlorophenyl)methyl]oxy}phenyl)methyl]-5-methyl-1 H-pyrazole-3-carboxylic acid |
| | | NMR | ¹H NMR δ: 2.18 (3H, s), 5.21 (2H, s), 5.44 (2H, s), 6.65 (1 H, s), 6.77 (1 H, d, J=7.0Hz), 6.91 - 6.98 (2H, m), 7.26 - 7.32 (3H, m), 7.43 - 7.49 (2H, m) |
| | | LCMS | t=3.38, [MH+] 357, 359, [MH-] 355, 357 |
| | | Method | E and Method 4 |
| 38 | 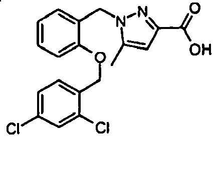 | Name | 1-[(2-{[(2,4-dichlorophenyl)methyl]oxylphenyl)methyl]-5-methyl-1 H-pyrazole-3-carboxylic acid |
| | | NMR | ¹H NMR δ: 2.19 (3H, s), 5.17 (2H, s), 5.44 (2H, s), 6.66 (1 H, s), 6.76 (1H, d, *J*=7.0Hz), 6.91 - 6.95 (2H, m), 7.25 - 7.31 (2H, m), 7.42 - 7.46 (2H, m) |
| | | LCMS | t=3.58, [MH+] 391, 393, [MH-] 389, 391 |
| | | Method | E and Method 4 |
| 39 | 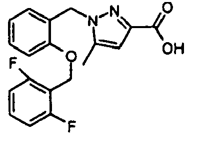 | Name | 1-[(2-{[(2,6-difluorophenyl)methyl]oxy}phenyl)methyl]-5-methyl-1 H-pyrazole-3-carboxylic acid |
| | | NMR | ¹H NMR δ: 2.12 (3H, s), 5.18 (2H, s), 5.32 (2H, s), 6.61 (1 H, s), 6.80 (1 H, d, *J*=6.5Hz), 6.91 - 6.99 (3H, m), 7.08 (1 H, d, J=8.3Hz), 7.29 - 7.39 (2H, m) |
| | | LCMS | t=3.18, [MH+] 359, [MH-] 357 |
| | | Method | E and Method 4 |
| 40 | 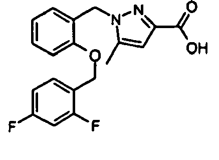 | Name | 1-[(2-{[(2,4-difluorophenyl)methyl]oxy)phenyl)methyl]-5-methyl-1 H-pyrazole-3-carboxylic acid |
| | | NMR | ¹H NMR δ: 2.10 (3H, s), 5.16 (2H, s), 5.23 (2H, s), 6.46 (1 H, s), 6.79 (1 H, d, J=7.5Hz), 6.92 - 6.95 (1 H, m), 7.09 - 7.18 (2H, m), 7.29 - 7.34 (2H, m), 7.61 - 7.67 (1 H, m) |
| | | LCMS | t=3.25, [MH+] 359, [MH-] 357 |
| | | Method | E and Method 4 |
| 41 | 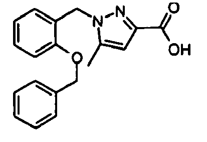 | Name | 5-methyl-1-({2-[(phenylmethyl)oxy]phenyl}methyl)-1*H*-pyrazole-3-carboxylic acid |
| | | NMR | ¹H NMR δ: 2.15 (3H, s), 5.11 (2H, s), 5.41 (2H, s), 6.65 (1 H, s), 6.78 (1 H, d, *J*=7.5Hz), 6.89 - 6.93 (1 H, m), 6.97 (1 H, d, J=8.3Hz), 7.24 - 7.28 (1 H, m), 7.36 - 7.42 (5H, m) |
| | | LCMS | t=3.19, [MH+] 323, [MH-] 321 |
| | | Method | E and Method 4 |
| 42 | 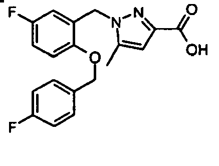 | Name | 1-[(5-fluoro-2-{[(4-fluorophenyl)methyl]oxy}phenyl)methyl]-5-methyl-1*H*-pyrazole-3-carboxylic acid |
| | | NMR | ¹H NMR δ: 2.17 (3H, s), 5.04 (2H, s), 5.34 (2H, s), 6.46 (1 H, dd, J=3.0Hz and 8.8Hz), 6.67 (1 H, s), 6.71 (1 H, d, *J*= 2.5Hz), 6.87 - 6.90 (1 H, m), 6.93 - 6.98 (1H, m), 7.08 - 7.12 (2H, m), 7.35 - 7.39 (2H, m) |
| | | LCMS | t=3.23, [MH+] 359, [MH-] 357 |
| | | Method | F and Method 4 |
| 43 | 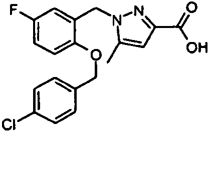 | Name | 1-[(2-{(4-chlorophenyl)methyl]oxy}-5-fluorophenyl)methyl]-5-methyl-1H-pyrazole-3-carboxylic acid |
| | | NMR | ¹H NMR δ: 2.18 (3H, s), 5.05 (2H, s), 5.37 (2H, s), 6.42 (1H, dd, J=3.0 and 8.5Hz), 6.67 (1H, s), 6.83 - 6.87 (1 H, m), 6.90 - 6.95 (1 H, m), 7.32 - 7.39 (4H, m) |
| | | LCMS | t=3.38, [MH+] 375, 377, [MH-] 373, 375 |
| | | Method | F and Method 4 |
| 44 | 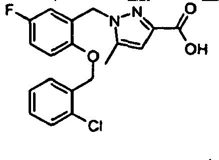 | Name | 1-[(2-{[(2-chlorophenyl)methyl]oxy}-5-fluorophenyl)methyl]-5-methyl-1H-pyrazole-3-carboxylic acid |
| | | NMR | ¹H NMR δ: 2.14 (3H, s), 5.21 (2H, s), 5.34 (2H, s), 6.49 (1 H, dd, J=2.8 and 8.8Hz), 6.56 (1H, s), 6.98 - 7.08 (2H, m), 7.31 - 7.36 (2H, m), 7.44 - 7.47 (1H, m), 7.49 - 7.51 (1H, m) |
| | | LCMS | t=3.39, [MH+] 375, 377, [MH-] 373, 375 |
| | | Method | F and Method 4 |
| 45 | 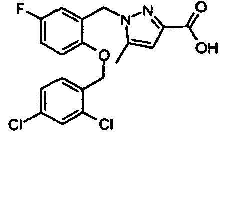 | Name | 1-[(2-{[(2,4-dichlorophenyl)methyl]oxy}-5-fluorophenyl)methyl]-5-methyl-1H-pyrazole-3-carboxylic acid |
| | | NMR | ¹H NMR δ: 2.13 (3H, s), 5.18 (2H, s), 5.28 (2H, s), 6.48 (1H, s), 6.59 (1 H, d, J=8.8Hz), 7.16 (2H, d, J=5.0Hz), 7.46 (1H, dd, J=8.3 and 2.0Hz), 7.64 (1H, d, J=8.3Hz), 7.71 (1 H, d, *J*=2.0Hz) |
| | | LCMS | t=3.59, [MH+] 409, 411, [MH-] 407, 409 |
| | | Method | F and Method 4 |
| 46 | 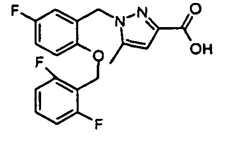 | Name | 1-[(2-{((2,6-difluorophenyl)methyl]oxy}-5-fluorophenyl)methyl]-5-methyl-1*H*-pyrazole-3-carboxylic acid |
| | | NMR | ¹H NMR δ: 2.13 (3H, s), 5.16 (2H, s), 5.30 (2H, s), 6.46 (1 H, dd, *J*=8.8 and 3.0Hz), 6.64 (1H, s), 6.95 - 7.04 (4H, m), 7.34 - 7.41 (1 H, m) |
| | | LCMS | t=3.21, [MH+] 377, [MH-] 375 |
| | | Method | F and Method 4 |
| 47 | 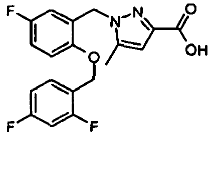 | Name | 1-[(2-{[(2,4-difluorophenyl)methyl]oxy}-5-fluorophenyl)methyl]-5-methyl-1 *H*-pyrazole-3-carboxylic acid |
| | | NMR | ¹H NMR δ: 2.18 (3H, s), 5.09 (2H, s), 5.35 (2H, s), 6.45 (1 H, dd, J=8.8 and 2.8Hz), 6.67 (1 H, s), 6.86 - 6.98 (4H, m), 7.38 - 7.44 (1 H, m) |
| | | LCMS | t=3.27, [MH+] 377, [MH-] 375 |
| | | Method | F and Method 4 |
| 48 | 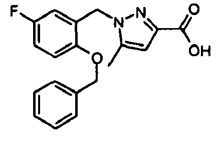 | Name | 1-({5-fluoro-2-[(phenylmethyl)oxy]phenyl}methyl)-5-methyl-1H-pyrazole-3-carboxylic acid |
| | | NMR | ¹H NMR δ: 2.17 (3H, s), 5.08 (2H, s), 5.37 (2H, s), 6.44 (1H, dd, J=8.8 and 2.8Hz), 6.67 (1H, s), 6.87 - 6.96 (2H, m), 7.34 - 7.43 (5H, m) |
| | | LCMS | t=3.22, [MH+] 341, [MH-] 339 |
| | | Method | F and Method 4 |

The intermediate 1,1-dimethylethyl 2-[(5-chloro-2-hydroxyphenyl)ethyl] - hydrazinecarboxylate was prepared from the appropriate ketone according to Method 3. 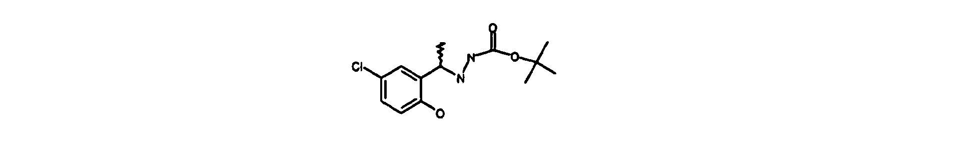 ¹H NMR (CDCl₃) δ: 1.41 (3H, d, J=6.8Hz), 1.48 (9H, s), 4.21-4.25 (1H, m), 6.23 (1H, br s), 6.77 (1H, d, J = 8.6Hz), 6.96 (1 H, d, J = 2.4Hz), 7.11(1H, dd, J=8.6 J=2.3 Hz).

The intermediate 1-[1-(5-chloro-2-hydroxy-phenyl]-ethyl]-5-methyl-1H-pyrazole-3-carboxylic acid ethyl ester (G) was prepared from 1,1-dimethlethyl 2-[(5-chloro-2-hydroxyphenyl)ethyl]hydrazinecarboxylate according to Method 3. 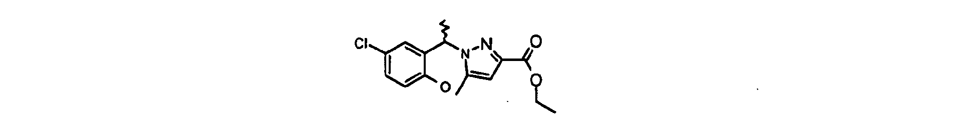 ¹H NMR (CDCl₃) δ: 1.28 (3H, t, J=7.1 Hz), 1.71 (3H, d, J= 6.8 Hz), 2.20 (3H, s), 4.25 (2H, dq, J=2.08 J= 7.1 Hz), 5.81 (1H, q, J=6.8 Hz), 6.56 (1H, s), 6.77 (1 H, d, J=2.6 Hz), 6.84 (1H, d, J=8.6 Hz), 7.14 (1H, dd, J=2.6 J=8.6 Hz), 10.15 (1 H, s).

### General Method 5

### 1-{1 [5-chloro-2-(2-chloro-4-fluoro-benzyloxy)-phenyl]-ethyl}-5-methyl-1H-pyrazole-3-carboxylic acid ethyl ester

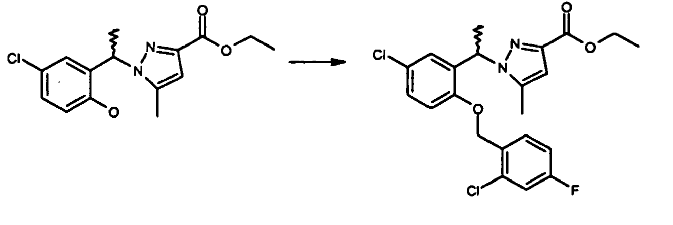

A mixture of 1-[1-(5-chloro-2-hydroxy-phenyl]-ethyl]-5-methyl-1H-pyrazole-3-carboxylic acid ethyl ester (100mg, 0.32mmol), K₂CO₃ (112mg, 0.81mmol) and 2-chloro-4-fluorobenzyl bromide (79mg, 0.36mmol) in acetone (3ml) was refluxed overnight under nitrogen. After cooling the solid was filtered off and the solvent removed in vacuo. Purification was carried out on a SPE using iso-hexane containing a gradient of ethyl acetate (5-10%) to yield the title compound (120mg, 74%).
t = 3.95, [MH+] 451,454.

The following 1H-pyrazole-3-carboxylic acid esters were prepared from G according to Method 5

| | | |
|---|---|---|
| 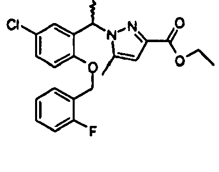 | Name | 1-{5-chloro-2-[(2-fluorobenzyloxy)-phenyl]-ethyl}-5-methyl-1*H*-pyrazole-3-carboxylic acid ethyl ester |
| | LCMS | t = 3.78, [MH+] 417,419 |
| | Method | G and Method 5 |
| 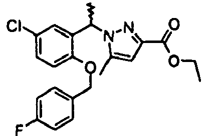 | Name | 1-{5-chloro-2-[(4-fluorobenzyloxy)-phenyl]-ethyl}-5-methyl-1*H*-pyrazole-3-carboxylic acid ethyl ester |
| | LCMS | t = 3.77, [MH+] 417,419 |
| | Method | G and Method 5 |
| 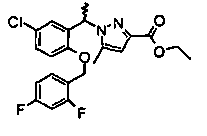 | Name | 1-{5-chloro-2-[(2,4-difluorobenzyloxy)-phenyl]-ethyl}-5-methyl-1*H*-pyrazole-3-carboxylic acid ethyl ester |
| | LCMS | t = 3.80, [MH+] 435,437 [MH-] 433 |
| | Method | G and Method 5 |
| 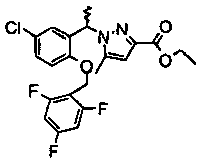 | Name | 1-{5-chloro-2-[(2,4,6-trifluorobenzyloxy)-phenyl]-ethyl}-5-methyl-1*H*-pyrazole-3-carboxylic acid ethyl ester |
| | LCMS | t = 3.58, [MH+] 453,455 |
| | Method | G and Method 5 |
| 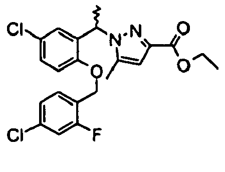 | Name | 1-{5-chloro-2-[(4-chloro-2-fluorobenzyloxy)-phenyl]-ethyl}-5-methyl-1H-pyrazole-3-carboxylic acid ethyl ester |
| | LCMS | t = 3.96, [MH+] 451,454 |
| | Method | G and Method 5 |
| 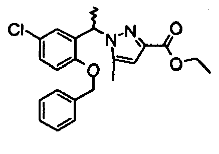 | Name | 1-{5-chloro-2-[(benzyloxy)-phenyl]-ethyl}-5-methyl-1 H-pyrazole-3-carboxylic acid ethyl ester |
| | LCMS | t = 3.77, [MH+] 399,401 |
| | Method | G and Method 5 |

### Preparation of 1-[1-(5-chloro-2-isobutoxy-phenyl)-ethyl]-5-methyl-1H-pyrazole-3-carboxylic acid ethyl ester

A mixture of 1-[1-(5-chloro-2-hydroxy-phenyl]-ethyl]-5-methyl-1*H*-pyrazole-3-carboxylic acid ethyl ester (100mg, 0.32mmol), K₂CO₃ (112mg, 0.81 mmol) and 1-bromo-2-methylpropane (0.038ml, 0.36mmol) in DMF (3ml) was heated at 80°C under nitrogen for 2 hours. After cooling the solution was diluted with water and extracted with ethyl acetate (3 x 10ml). The combined extracts were dried (MgSO₄) and evaporated. Purification was carried out on a SPE (20% ethyl acetate :iso-hexane) to yield the title compound.
t = 3.88, [MH+] 365, 367.

### General Method 6

### Example 49: 1-{1-[5-chloro-2-(2-chloro-4-fluoro-benzyloxy)-phenyl]-ethyl}-5-methyl-1H-pyrazole-3-carboxylic acid

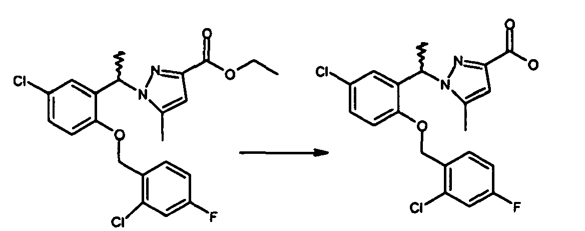

To a solution of 1-{1-[5-chloro-2-(2-chloro-4-fluoro-benzyloxy)-phenyl]-ethyl}-5-methyl-1H-pyrazole-3-carboxylic acid ethyl ester (120mg, 0.26mmol) in 3 ml of ethanol and 1ml of water, NaOH (42mg, 1.06mmol) was added. The mixture was stirred at 60°C for 2 hours. the solution was diluted with water, acidified with acetic acid and extracted with ethyl acetate. The organic solution was dried over MgSO₄ and evaporated to give the title compound (112mg, 99%).
¹H NMR (DMSO) δ: 1.67 (3H, bs), 1.94 (3H, s), 5.21 (2H, s), 5.66 (1H, q, J=6.8 Hz), 6.12 (1H, s), 6.82 (1 H, d, J=2.6 Hz), 7.18 (1H, d, J=8.8 Hz), 7.19-7.31 (2H, m), 7.56 (1H, dd, J=2.6 J=8.8 Hz), 7.64 (1 H, m).
t = 3.76, [MH-] 421, 424.

The following Examples were prepared from the appropriate ester intermediate according to Method 6

| | | | |
|---|---|---|---|
| 50 | 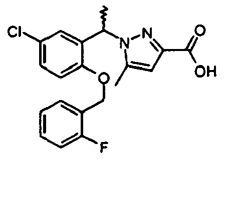 | Name | 1-{5-chloro-2-[(2-fluorobenzyloxy)-phenyl]-ethyl}-5-methyl-1*H*-pyrazole-3-carboxylic acid |
| | | NMR | ¹H NMR δ: (DMSO) 1.68 (3H, d, J=6.8 Hz), 2.01 (3H, s), 5.17-5.24 (2H, m), 5.77 (1 H, q, J=6.8 Hz), 6.43 (1H, s), 6.93 (1 H, d, J=2.6 Hz), 7.2-7.5 (6H, m), 12.6 (1H,bs). |
| | | LCMS | t = 3.59, [MH+] 389 [MH-] 387, 389 |
| | | Method | Method 6 |
| 51 | 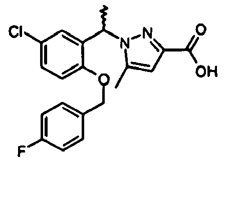 | Name | 1-{5-chloro-2-[(4-fluorobenzyloxy)-phenyl]-ethyl}-5-methyl-1*H*-pyrazole-3-carboxylic acid |
| | | NMR | ¹H NMR δ: (DMSO) 1.68 (3H, d, J=6.8 Hz), 2.0 (3H, s), 5.17 (2H, s), 5.73 (1H, q, J=6.8 Hz), 6.26 (1 H, s), 6.88 (1 H, d, J=2.6 Hz), 7.13 (1 H, d, J= 8.8 Hz), 7.12-7.32 (3H, m), 7.46-7.5 (2H, m). |
| | | LCMS | t = 3.56, [MH+] 3.89 [MH-] 387, 389 |
| | | Method | Method 6 |
| 52 | 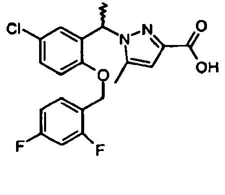 | Name | 1-{5-chloro-2-[(2,4-difluorobenzyloxy)-phenyl]-ethyl}-5-methyl-1H-pyrazole-3-carboxylic acid |
| | | NMR | ¹H NMR δ: (DMSO) 1.66 (3H, d, J=6.8 Hz), 2.0 (3H, s), 5.2 (2H, s), 5.63 (1 H, q, J=6.8 Hz), 6.14 (1H, s), 6.82 (1H, d, J=2.6 Hz), 7.12-7.21 (2H, m), 7.28-7.37 (2H,m), 7.60 (1H, q, J=6.8 Hz). |
| | | LCMS | t = 3.61, [MH+] 407, 409 [MH-] 405, 407 |
| | | Method | Method 6 |
| 53 | 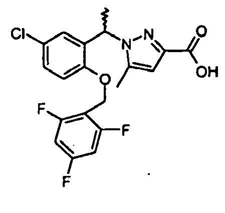 | Name | 1-{5-chloro-2-[(2,4,6-trifluorobenzyloxy)-phenyl]-ethyl}-5-methyl-1H-pyrazole-3-carboxylic acid |
| | | NMR | ¹H NMR δ: (DMSO) 1.63 (3H, d, J=6.8 Hz), 1.96 (3H, s), 5.14 (2H, q, J=11 Hz), 5.65 (1 H, q, J=6.8 Hz), 6.37 (1H, s), 6.96 (1H, s), 7.25-7.39 (4H, m). |
| | | LCMS | t = 3.58, [MH+] 425,427 [MH-] 423,425 |
| | | Method | Method 6 |
| 54 | 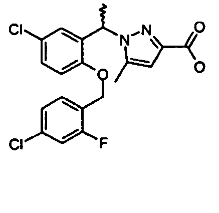 | Name | 1-{5-chloro-2-[(4-chloro-2-fluorobenzyloxy)-phenyl]-ethyl}-5-methyl-1 H-pyrazole-3-carboxylic acid |
| | | NMR | ¹H NMR δ: (DMSO) 1.68 (3H, d, J=6.8 Hz), 1.97 (3H, s), 5.21 (2H, s), 5.67 (1 H, q, J=6.8 Hz), 6.18 (1H, s), 6.82 (1H, d, J=2.6 Hz), 7.17 (1 H, d, J=8.8 Hz), 7.24-7.38 (2H, m), 7.50-7.62 (2H, m). |
| | | LCMS | t = 3.83, [MH-] 421 |
| | | Method | Method 6 |
| 55 | 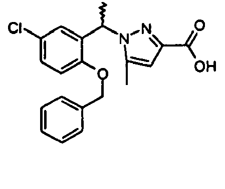 | Name | 1-{5-chloro-2-[(benzyloxy)-phenyl]-ethyl}-5-methyl-1 H-pyrazole-3-carboxylic acid |
| | | NMR | ¹H NMR δ: (DMSO) 1.68 (3H, bs), 2.1 (3H, s), 5.21 (2H, s), 5.75 (1 H, q, J=6.8 Hz), 6.2 (1 H, s), 6.82 (1H, d, J=2.6 Hz), 7.15 (1H, d, J=8.8 Hz), 7.21-7.5 (6H, m). |
| | | LCMS | t = 3.58, [MH+] 371 |
| | | Method | Method 6 |
| 56 | 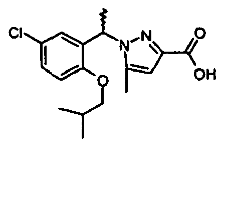 | Name | 1-[1-(5-chloro-2-isobutoxy-phenyl)-ethyl]-5-methyl-1*H*-pyrazole-3-carboxylic acid |
| | | NMR | ¹H NMR δ: (DMSO) 0.98 (6H, t, J=7.08 Hz), 1.71 (3H, d, J=6.88 Hz), 2.02-2.06 (1H, m), 2.18 (3H, s), 3.80 (2H, d, J=6.36 Hz), 5.83-5.87(1 H, m), 6.52 (1H, s), 6.85 (1H, d, J=2.6 Hz), 7.04 (1H, d, J=8.8 Hz), 7.3 (1H, dd, J=8.7, J=2.6 Hz), 12.6 (1 H, bs). |
| | | LCMS | t = 3.65, [MH+] 337 |
| | | Method | Method 6 |

### Preparation of 1-(5-chloro-2-hydroxy-benzyl)-5-methyl-1H-pyrrole-3-carboxylic acid ethyl ester (H)

### Preparation of 2-benzvloxy-5-chloro-benzamide

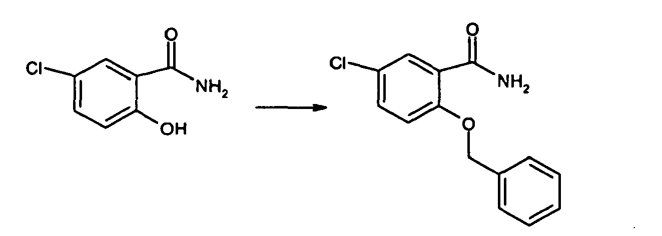

A mixture of 5-chloro-2-hydroxy-benzamide (8g, 0.046mol), K₂CO₃ (7.72g, 0.056mol) and benzyl bromide (6.1 ml, 0.051 mol) in acetone (50ml) was refluxed overnight, under nitrogen. After cooling, the solid was filtered off and the filtrate was cooled (in a fridge) to effect crystallisation. The resultant solid was collected to give 9.9g (81 %) of a colourless solid.
t = 2.90, [MH+] 262, 264

### Preparation of 2-benzyloxy-5-chloro-benzylamine

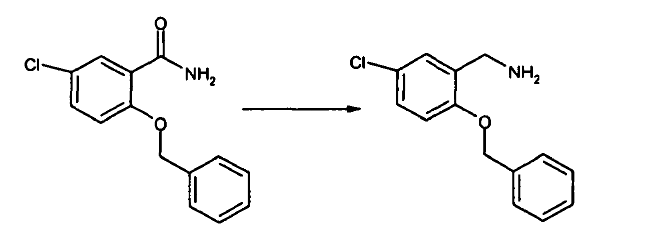

2-benzyloxy-5-chloro-benzamide (7.9g, 0.030mol) in 20ml of tetrahydrofuran was slowly added, under nitrogen, to a 1 M solution of LiAlH₄ (45ml) in tetrahydrofuran at 0°C. The reaction mixture was then heated at 70°C for 1 hour. After cooling the reaction mixture was poured onto water and extracted with ethyl acetate (3 x 40ml). The combined extracts were dried (MgSO₄) and evaporated to give the title compound as a yellow oil (7g, 94%).
¹H NMR δ: 1.66 (2H, bs), 3.84 (2H, s), 5.07 (2H, s), 6.83 (1H, d, J=8.6Hz), 7.15 (1H, dd, J=8.6 and 2.6Hz), 7.24-7.42 (6H,m).

### Preparation of 4,4-dimethoxy-pentanoic acid methyl ester

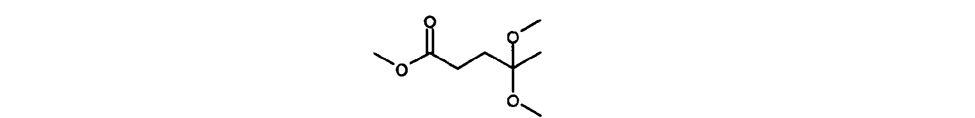

Ethyl levulinate (20g, 0.138mol), trimethyl orthoformate (15.3g, 0.144mol) and a catalytic amount of *p*-toluene sulfonic acid monohydrate in 6 ml of methanol were refluxed over the weekend. After cooling the mixture was vacum down and the residue used with no further purifications.
¹H NMR δ: 1.25 (3H, bs), 1.94-1.98 (2H, m), 2.32-2.37 (2H,m), 3.17 (6H, s), 3.68 (3H, s).

### Preparation of 2-formyl-4-oxo-pentanoic acid ethyl ester

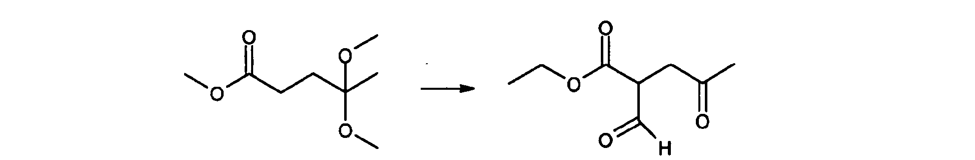

A mixture of 4,4-dimethoxy-pentanoic acid methyl ester (25g, 0.13mol) and ethyl formate (21ml, 0.26mol), was added to a solution of NaH (5.78g, 0.144mol) in THF (50ml) at -10°C. The reaction mixture was stirred for 3h, then let stand overnight. Water (100ml) and ether(60ml) were added and the mixture stirred for 5 minutes. The organic phase was then separated and washed with water. The combined water layers were acidified to pH2 and extracted with ethyl acetate (3x50ml). The combined extracts were dried (MgSO₄) and evaporated. The residue was then distilled, the fraction with b.p. 110-120°C was the desired compound.
¹H NMR δ: 1.27-1.32 (3H, m), 2.23 (3H, s), 2.63 (1H, t, J=6.7Hz), 2.76 (1H, t, J=6.7Hz), 3.78-3.81 (1H, m), 4.19-4.28 (2H, m), 9.93 (1H, s).

### Preparation of 1-(2-benzyloxy-5-chloro-benzyl)-5-methyl-1H-pyrrole-3 carboxylic acid ethyl ester

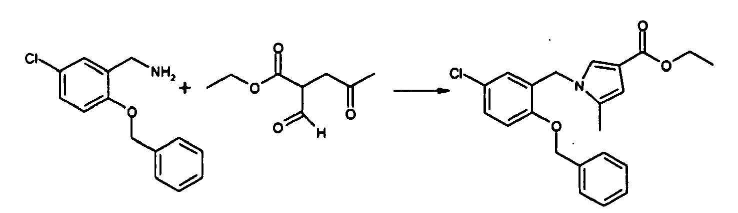

To a mixture of 2-formyl-4-oxo-pentanoic acid ethyl ester (2.5g, 0.016mol) and 2-benzyloxy-5-chloro-benzylamine (4.7g, 0.019mol), CH₃COOH (~3ml) was added. The reaction mixture was stirred for 2 hours then was poured onto water and extracted with ethyl acetate (3 x 40ml). The combined extracts were dried (MgSO₄) and evaporated. The residue was purified on a Biotage (15% ethyl acetate:iso-hexane) to give the title compound as a yellow solid (2.8g, 45%).
¹H NMR δ: 1.32 (3H, t, J=7.1 Hz), 2.08 (3H, s), 4.25 (2H, q, J=7.1 Hz), 4.98 (2H, s), 5.07 (2H, s), 6.35 (1H, s), 6.61 (1 H, s), 6.87 (1H, d, J=8.7Hz), 7.18-7.21 (2H, m), 7.33-7.41 (5H, m).

### Preparation of 1-(5-chloro-2-hydroxy-benzyl)-5-methyl-1H-pyrrole-3-carboxylic acid

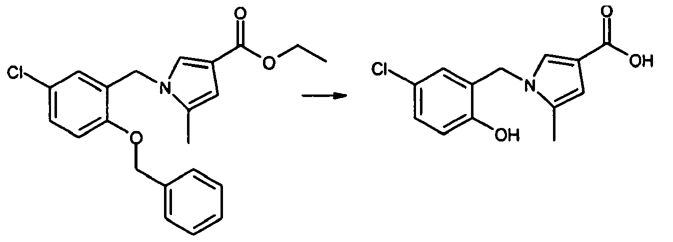

A mixture of sodium methanethiolate (1.16g, 16.5mmol) and 1-(2-benzyloxy-5-chlorobenzyl)-5-methyl-1*H*-pyrrole-3 carboxylic acid ethyl ester (1.27g, 3.3mmol) in DMF(14ml) was stirred at 100°C for 3 hours. After cooling the mixture was diluted with water and acidified with 1 M HCl and then extracted with ethyl acetate. The organic phase was dried (MgSO₄), evaporated to dryness to give the title compound as a yellow oil.
t = 2.76, [MH+] 266 [MH-] 264.

### Preparation of 1-(5-chloro-2-hydroxy-benzyl)-5-methyl-1H-pyrrole-3-carboxylic acid ethyl ester

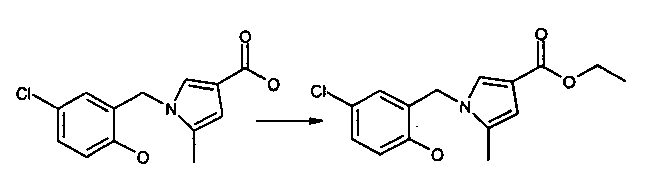

A mixture of 1-(5-chloro-2-hydroxy-benzyl)-5-methyl-1*H*-pyrrole-3-carboxylic acid (3.3mmol) and H₂SO₄ (1.5ml) in ethanol (15ml) was refluxed overnight.
After cooling the mixture was diluted with water basified with NH₃ and then extracted with ethyl acetate (3x20ml). The combined organic layers were dried (MgSO₄), and the solvent removed *in vacuo.* Purification was carried out on a SPE using 30% ethyl acetate in iso-hexane to yield the title compound as a yellow solid (0.73g,75%).
t = 3.28, [MH+] 294,296 [MH-] 292.

The following intermediates were prepared from 1-(5-chloro-2-hydroxy-benzyl)-5-methyl-1 H-pyrrole-3-carboxylic acid ethyl ester (intermediate H) according to Method 5.

| | | |
|---|---|---|
| 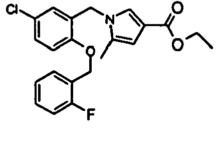 | Name | 1-[5-chloro-2-(2-fluoro-benzyloxy)-benzyl]-5-methyl-1H-pyrrole-3-carboxylic acid ethyl ester |
| | LCMS | t = 3.92, [MH+] 402,404 |
| | Method | H and Method 5 |
| 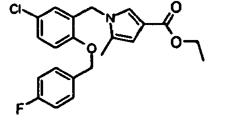 | Name | 1-[5-chloro-2-(4-fluoro-benzyloxy)-benzyl]-5-methyl-1H-pyrrole-3-carboxylic acid ethyl ester |
| | LCMS | t = 3.91, [MH+] 402,404 |
| | Method | H and Method 5 |
| 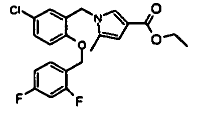 | Name | 1-[5-chloro-2-(2,4-difluoro-benzyloxy)-benzyl]-5-methyl-1H-pyrrole-3-carboxylic acid ethyl ester |
| | LCMS | t = 3.93, [MH+] 420,422 |
| | Method | H and Method 5 |
| 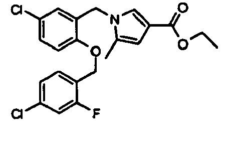 | Name | 1-[5-chloro-2-(4-chloro-2-fluoro-benzyloxy)-benzyl]-5-methyl-1H-pyrrole-3-carboxylic acid ethyl ester |
| | LCMS | t = 4.09, [MH+] 436,439 |
| | Method | H and Method 5 |
| 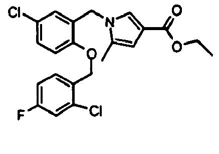 | Name | 1-[5-chloro-2-(2-chloro-4-fluoro-benzyloxy)-benzyl]-5-methyl-1*H*-pyrrole-3-carboxylic acid ethyl ester |
| | LCMS | t = 4.08, [MH+] 436,439 |
| | Method | H and Method 5 |

The following Examples were prepared from the appropriate ester intermediate according to Method 6.

| | | | |
|---|---|---|---|
| 57 | 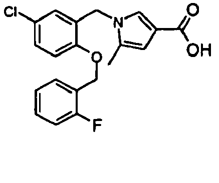 | Name | 1-[5-chloro-2-(2-fluoro-benzyloxy)-benzyl]-5-methyl-1H-pyrrole-3-carboxylic acid |
| | | NMR | ¹H NMR (DMSO)δ: 1.99 (3H, s), 5.03 (2H, s), 5.23 (2H, s), 6.15 (1H, s), 6.62 (1H, s), 7.21-7.29 (5H, m), 7.36 (1 H, d, J=8 Hz), 7.41-7.45 (1H, m), 7.53 (1H, t, J=7.4 Hz), 11.6 (1H, s). |
| | | LCMS | t = 3.50, [MH+] 374,376 [MH-] 372,374 |
| | | Method | Method 6 |
| 58 | 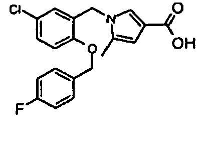 | Name | 1-[5-chloro-2-(4-fluoro-benzyloxy)-benzyl]-5-methyl-1H-pyrrole-3-carboxylic acid |
| | | NMR | ¹H NMR (DMSO)δ: 2.03 (3H, s), 5.07 (2H, s), 5.17 (2H, s), 6.17 (1H, s), 6.64 (1H, s), 7.14-7.23 (3H, m), 7.29 (1H, s), 7.33 (1 H, bd), 7.47-7.5 (2H, m), 11.59 (1 H, s). |
| | | LCMS | t = 3.48, [MH+] 374,376 [MH-] 372,374 |
| | | Method | Method 6 |
| 59 | 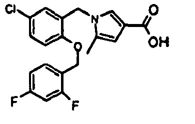 | Name | 1-[5-chloro-2-(2,4-difluoro-benzyloxy)-benzyl]-5-methyl-1 H-pyrrole-3-carboxylic acid |
| | | NMR | ¹H NMR (DMSO)δ: 1.99 (3H, s), 5.02 (2H, s), 5.19 (2H, s), 6.15 (1H, s), 6.64 (1H, s), 7.08-7.14 (1H, m), 7.21-7.38 (4H, m), 7.58-7.65 (1H, m), 11.6 (1 H, s). |
| | | LCMS | t = 3.48, [MH+] 392,394 [MH-] 390,392 |
| | | Method | Method 6 |
| 60 | 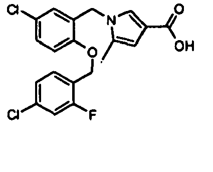 | Name | 1-[5-chloro-2-(4-chloro-2-fluoro-benzyloxy)-benzyl]-5-methyl-1H-pyrrole-3-carboxylic acid |
| | | NMR | ¹H NMR (DMSO)δ: 2.0 (3H, s), 5.03 (2H, s), 5.21 (2H, s), 6.16 (1 H, s), 6.63 (1 H, s), 7.21 (1H, d, J=8.8 Hz), 7.28 (1H, s), 7.31-7.37 (2H, m), 7.5-7.58 (2H, m), 11.6 (1H, s). |
| | | LCMS | t = 3.70, [MH+] 408,411 [MH-] 406,410 |
| | | Method | Method 6 |
| 61 | 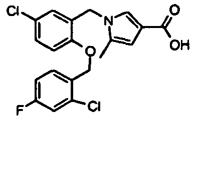 | Name | 1-[5-chloro-2-(2-chloro-4-fluoro-benzyloxy)-benzyl]-5-methyl-1H-pyrrole-3-carboxylic acid |
| | | NMR | ¹H NMR (DMSO)δ: 1.99 (3H, s), 5.06 (2H, s), 5.21 (2H, s), 6.16 (1 H, s), 6.63 (1 H, s), 7.19-7.28 (3H, m), 7.37 (1H, bd), 7.55 (1H, bd), 7.6-7.68 (1H, m), 11.6 (1H, s). |
| | | LCMS | t = 3.70, [MH+] 408,411 [MH-] 406,409 |
| | | Method | Method 6 |

It is to be understood that the present invention covers all combinations of particular and preferred subgroups described herein above.

### ASSAYS FOR DETERMINING BIOLOGICAL ACTIVITY

The compounds of formula (I) can be tested using the following assays to demonstrate their prostanoid antagonist or agonist activity in vitro and in vivo and their selectivity. The prostaglandin receptors investigated are DP, EP₁, EP₂, EP₃, EP₄, FP, IP and TP.

### Biological Activity at EP₁ and EP₃ Receptors

The ability of compounds to antagonise EP₁ & EP₃ receptors may be demonstrated using a functional calcium mobilisation assay. Briefly, the antagonist properties of compounds are assessed by their ability to inhibit the mobilisation of intracellular calcium ([Ca²+]ᵢ) in response to activation of EP₁ or EP₃ receptors by the natural agonist hormone prostaglandin E₂ (PGE₂). Increasing concentrations of antagonist reduce the amount of calcium that a given concentration of PGE₂ can mobilise. The net effect is to displace the PGE₂ concentration-effect curve to higher concentrations of PGE₂. The amount of calcium produced is assessed using a calcium-sensitive fluorescent dye such as Fluo-3, AM and a suitable instrument such as a Fluorimetric Imaging Plate Reader (FLIPR). Increasing amounts of [Ca²⁺]ᵢ produced by receptor activation increase the amount of fluorescence produced by the dye and give rise to an increasing signal. The signal may be detected using the FLIPR instrument and the data generated may be analysed with suitable curve-fitting software.

The human EP₁ or EP₃ calcium mobilisation assay (hereafter referred to as 'the calcium assay') utilises Chinese hamster ovary-K1 (CHO-K1) cells into which a stable vector containing either EP₁ or EP₃ cDNA has previously been transfected. Cells are cultured in suitable flasks containing culture medium such as DMEM:F-12 supplemented with 10% v/v foetal calf serum, 2mM L-glutamine, 0.25mg/ml geneticin and 10µg/ml puromycin.

For assay, cells are harvested using a proprietary reagent that dislodges cells such as Versene. Cells are re-suspended in a suitable quantity of fresh culture media for introduction into a 384-well plate. Following incubation for 24 hours at 37°C the culture media is replaced with a medium containing fluo-3 and the detergent pluronic acid, and a further incubation takes place. Concentrations of compounds are then added to the plate in order to construct concentration-effect curves. This may be performed on the FLIPR in order to assess the agonist properties of the compounds. Concentrations of PGE₂ are then added to the plate in order to assess the antagonist properties of the compounds.

The data so generated may be analysed by means of a computerised curve-fitting routine. The concentration of compound that elicits a half-maximal inhibition of the calcium mobilisation induced by PGE₂ (pIC₅₀) may then be estimated.

### Binding Assay for the Human Prostanoid EP₁ Receptor

Competition assay using [³H]-PGE2.

Compound potencies are determined using a radioligand binding assay. In this assay compound potencies are determined from their ability to compete with tritiated prostaglandin E₂ ([³H]-PGE₂) for binding to the human EP₁ receptor.

This assay utilises Chinese hamster ovary-K1 (CHO-K1) cells into which a stable vector containing the EP₁ cDNA has previously been transfected. Cells are cultured in suitable flasks containing culture medium such as DMEM:F-12 supplemented with 10% v/v foetal calf serum, 2mM L-glutamine, 0.25mg/ml geneticin, 10µg/ml puromycin and 10µM indomethacin.

Cells are detached from the culture flasks by incubation in calcium and magnesium free phosphate buffered saline containing 1 mM disodium ethylenediaminetetraacetic acid (Na₂EDTA) and 10µM indomethacin for 5 min. The cells are isolated by centrifugation at 250xg for 5mins and suspended in an ice cold buffer such as 50 mM Tris, 1 mM Na₂EDTA, 140mM NaCl, 10µM indomethacin (pH 7.4). The cells are homogenised using a Polytron tissue disrupter (2x10s burst at full setting), centrifuged at 48,000xg for 20mins and the pellet containing the membrane fraction is washed three times by suspension and centrifugation at 48,000xg for 20mins. The final membrane pellet is suspended in an assay buffer such as 10mM 2-[N-morpholino]ethanesulphonic acid, 1 mM Na₂EDTA, 10mM MgCl₂ (pH 6). Aliquots are frozen at -80°C until required.

For the binding assay the cell membranes, competing compounds and [³H]-PGE₂ (3nM final assay concentration) are incubated in a final volume of 100µl for 30 min at 30°C. All reagents are prepared in assay buffer. Reactions are terminated by rapid vacuum filtration over GF/B filters using a Brandell cell harvester. The filters are washed with ice cold assay buffer, dried and the radioactivity retained on the filters is measured by liquid scintillation counting in Packard TopCount scintillation counter.

The data are analysed using non linear curve fitting techniques (GraphPad Prism 3) to determine the concentration of compound producing 50% inhibition of specific binding (IC₅₀).

### Biological Activity at TP Receptor

To determine if a compound has agonist or antagonist activity at the TP receptor a functional calcium mobilisation assay may be performed. Briefly, the antagonist properties of compounds are assessed by their ability to inhibit the mobilisation of intracellular calcium ([Ca²⁺]ᵢ) in response to activation of TP receptors by the stable TXA₂ mimetic U46619. Increasing concentrations of antagonist reduce the amount of calcium that a given concentration of U46619 can mobilise. The net effect is to displace the U46619 concentration-effect curve. The amount of calcium produced is assessed using a calcium-sensitive fluorescent dye such as Fluo-3, AM and a suitable instrument such as a Fluorimetric Imaging Plate Reader (FLIPR). Increasing amounts of [Ca²⁺]ᵢ produced by receptor activation increase the amount of fluorescence produced by the dye and give rise to an increasing signal. The signal may be detected using the FLIPR instrument and the data generated may be analysed with suitable curve-fitting software. The agonist activity of the compounds are determined by their ability to cause an increase in intracellular mobilisation in the absence of U46619.

The human TP calcium mobilisation assay utilises Chinese hamster ovary-K1 (CHO-K1) cells into which a stable vector containing TP cDNA has previously been transfected. Cells are cultured in suitable flasks containing culture medium such as DMEM:F-12 supplemented with 10% v/v foetal calf serum, 2mM L-glutamine, 0.25mg/ml geneticin and 10µg/ml puromycin.

For assay, cells are harvested using a proprietary reagent that dislodges cells such as Versene. Cells are re-suspended in a suitable quantity of fresh culture media for introduction into a 96-well plate. Following incubation for 24 hours at 37°C the culture media is replaced with a medium containing fluo-3 and the detergent pluronic acid, and a further incubation takes place. Concentrations of compounds are then added to the plate in order to construct concentration-effect curves. This may be performed on the FLIPR in order to assess the agonist properties of the compounds. Concentrations of U46619 are then added to the plate in order to assess the antagonist properties of the compounds.

The data so generated may be analysed by means of a computerised curve-fitting routine. The concentration of compound that elicits a half-maximal inhibition of the calcium mobilisation induced by U46619 (pIC₅₀) may then be estimated, and the percentage activation caused by the compounds directly can be used to determine if there is any agonism present.

By application of these techniques, compounds of the Examples had an antagonist binding plC₅₀ value of 6.2- 9.9 at EP₁ receptors and a plC50 value of < 5.7 at EP₃ receptors. The compounds of the examples had a functional pKi of 6.2-10.5 and/or a functional plC₅₀ of 5.3-8.9.

No toxicological effects are indicated/expected when a compound (of the invention) is administered in the above mentioned dosage range.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation the following claims:

## Claims

1. A compound of formula (I): 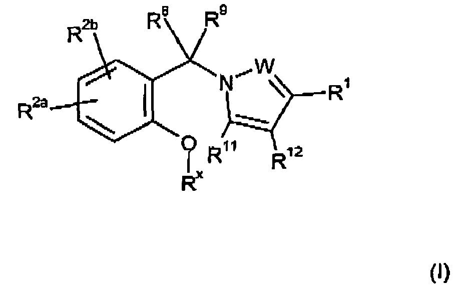 wherein:
W is N or CR¹⁰;
R¹ is CO₂H;
R^{2a} and R^{2b} are independently selected from hydrogen, halo, C₁₋₄alkyl, CF₃, and OC₁₋₆alkyl;
R^{x} is selected from CH₂-pyridyl, C₁₋₆alkyl or CH₂Ph wherein Ph is substituted by R^{3a}, R^{3b} and R^{3c};
R^{3a}, R^{3b} and R^{3c} are independently selected from hydrogen, halogen, NO₂, OCH₃, CH₃ and CF₃;
R⁸ is hydrogen;
R⁹ is hydrogen or CH₃;
R¹⁰ is selected from hydrogen, halogen, CH₃ and CF₃;
R¹¹ is selected from hydrogen, halogen, CH₃, CF₃ and thienyl; and
R¹² is selected from hydrogen, halogen, CH₃ and CF₃;
or a pharmaceutically acceptable salt or solvate thereof.

2. A compound according to claim 1 selected from:
1-({5-bromo-2-[{phenylmethyl)oxy]phenyl}methyl)5-methyl-1*H*-pyrazole-3-carboxylic acid;
1-({5-chloro-2-[(phenylmethyl)oxy]phenyl}methyl)-1*H*-pyrazole-3-carboxylic acid;
1-[(5-bromo-2-{[(2,4-difluorophenyl)methyl]oxy}phenyl)methyl]-5-methyl-1*H*-pyrazole-3-carboxylic acid;
1-({5-chloro-2-[(2-methylpropyl)oxy]phenyl}methyl)-5-methyl-1*H*-pyrazole-3-carboxylic acid;
1-{5-bromo-2-[(2,4-dichlorobenzyl)oxy]benzyl}-5-methyl-1H-pyrazole-3-carboxylic acid;
1-{5-bromo-2-[(4-chlorobenzyl)oxy]benzyl}-5-methyl-1H-pyrazole-3-carboxylic acid;
1-{5-bromo-2-[(4-fluorobenzyl)oxy]benzyl}-5-methyl-1H-pyrazole-3-carboxylic acid;
1-{5-bromo-2-[(2-chlorobenzyl)oxy]benzyl}-5-methyl-1H-pyrazole-3-carboxylic acid;
1-[2-(benzyloxy)-5-bromobenzyl]-1H-pyrazole-3-carboxylic acid;
1-{5-bromo-2-[(2-methoxybenzyl)oxy]benzyl}-5-methyl-1H-pyrazole-3-carboxylic acid;
1-(5-bromo-2-butoxybenzyl)-5-methyl-1H-pyrazole-3-carboxylic acid;
1-(5-bromo-2-{[4-(trifluoromethyl)benzyl]oxy}benzyl)-5-methyl-1H-pyrazole-3-carboxylic acid;
1-{5-bromo-2-[(2,6-difluorobenzyl)oxy]benzyl}-5-methyl-1*H*-pyrazole-3-carboxylic acid;
1-{5-bromo-2-[(3-bromobenzyl)oxy]benzyl}-5-methyl-1H-pyrazole-3-carboxylic acid;
1-{5-bromo-2-[(3-chlorobenzyl)oxy]benzyl}-5-methyl-1H-pyrazole-3-carboxylic acid;
1-[5-bromo-2-(pyridin-4-ylmethoxy)benzyl]-5-methyl-1H-pyrazole-3-carboxylic acid;
1-{5-bromo-2-[{3-methylbenzyl)oxy]benzyl}-5-methyl-1H-pyrazole-3-carboxylic acid;
1-{5-bromo-2-[(3-nitrobenzyl)oxy]benzyl}-5-methyl-1H-pyrazole-3-carboxylic acid;
1-[2-(benzyloxy)-5-bromobenzyl]-5-thien-2-yl-1H-pyrazole-3-carboxylic acid;
1-[2-{benzyloxy)-5-bromobenzyl]-4-fluoro-1H-pyrazole-3-carboxylic acid;
1-[(5-chloro-2-{[(4-fluorophenyl)methyl]oxy}phenyl)methyl]-5-methyl-1*H*-pyrazole-3-carboxylic acid;
1-[(5-chloro-2-{(4-chlorophenyl)methyl]oxy}phenyl)methyl]-5-methyl-1*H*-pyrazole-3-carboxylic acid;
1-[(5-chloro-2-{[(2-chlorophenyl)methyl]oxy}phenyl)methyl]-5-methyl-1*H*-pyrazole-3-carboxylic acid;
1-[(5-chloro-2-{[(2,4-dichlorophenyl)methy]oxy}phenyl)methy]-5-methyl-1*H*-pyrazole-3-carboxylic acid;
1-[(5-chloro-2-{[(2,6-difluorophenyl)methyl]oxy}phenyl)methyl]-5-methyl-1*H*-pyrazole-3-carboxylic acid;
1-[(5-chloro-2-{[(2,4-difluorophenyl)methyl]oxy}phenyl)methyl]-5-methyl-1*H*-pyrazole-3-carboxylic acid;
1-({5-chloro-2-[(phenylmethyl)oxy]phenyl}methyl)5-methyl-1*H*-pyrazole-3-carboxylic acid
1-{[2-{[(4-fluorophenyl)methyl]oxy}-5-(methyloxy)phenyl]methyl}-5-methyl-1*H*-pyrazole-3-carboxylic acid;
1-{[2-{[(4-chlorophenyl)methyl]oxy]-5-(methyloxy)phenyl]methyl}-5-methyl-1*H*-pyrazole-3-carboxylic acid;
1-{[2-{[(2-chlorophenyl)methyl]oxy}-5-(methyloxy)phenyl]methyl}-5-methyl-1*H*-pyrazole-3-carboxylic acid;
1-[[2-{[(2,4-dichlorophenyl)methyl]oxy}-5-(methyloxy)phenyl]methyl}-5-methyl-1*H*-pyrazole-3-carboxylic acid;
1-{[2-f[(2,6-difluorophenyl)methyl]oxy)-5-(methyloxy)phenyl]methyl}-5-methyl-1*H*-pyrazole-3-carboxylic acid;
1-{[2-{[(2,4-difluorophenyl)methyl]oxy}-5-(methyloxy)phenyl]methyl}-5-methyl-1*H*-pyrazole-3-carboxylic acid;
5-methyl-1-({5-(methyloxy)-2-[(phenylmethyl)oxy]phenyl}methyl)-1*H*-pyrazole-3-carboxylic acid;
1-[(2-{[(4-fluorophenyl)methyl]oxy}phenyl)methyl]-5-methyl-1*H*-pyrazole-3-carboxylic acid;
1-[(2-{[(4-chlorophenyl)methyl]oxy}phenyl)methyl]-5-methyl-1*H*-pyrazole-3-carboxylic acid;
1-[(2-{[(2-chlorophenyl)methyl]oxy}phenyl)methyl]-5-methyl-1*H*-pyrazole-3-carboxylic acid;
1-[(2-{[(2,4-dichlorophenyl)methyl]oxy}phenyl)methyl]-5-methyl-1*H*-pyrazole-3-carboxylic acid;
1-[(2-{[(2,6-difluorophenyl)methyl]oxy}phenyl)methyl]-5-methyl-1*H*-pyrazole-3-carboxylic acid;
1-[(2-{[(2,4-difluorophenyl)methyl]oxy}phenyl)methyl]-5-methyl-1*H*-pyrazole-3-carboxylic acid;
5-methyl-1-({2-[(phenylmethyl)oxy]phenyl}methyl)-1H-pyrazole-3-carboxylic acid
1-[(5-fluoro-2-{[(4-fluorophenyl)methyl]oxy}phenyl)methyl]-5-methyl-1*H*-pyrazole-3-carboxylic acid;
1-[(2-{[(4-chlorophenyl)methyl]oxy}-5-fluorophenyl)methyl]-5-methyl-1*H*-pyrazole-3-carboxylic acid;
1-[{2-{[(2-chlorophenyl)methyl]oxy}-5-fluorophenyl)methyl]-5-methyl-1*H*-pyrazole-3-carboxylic acid;
1-[(2-{[(2,4-dichlorophenyl)methyl]oxy}-5-fluorophenyl)methyl]-5-methyl-1*H*-pyrazole-3-carboxylic acid;
1-[(2-{[(2,6-difluorophenyl)methyl]oxy}-5-fluorophenyl)methyl]-5-methyl-1H-pyrazole-3-carboxylic acid;
1-[(2-{[(2,4-difluorophenyl)methyl]oxy)-5-fluorophenyl)methyl]-5-methyl-1*H*-pyrazole-3-carboxylic acid;
1-({5-fluoro-2-[(phenylmethyl)oxy]phenyl}methyl)-5-methyl-1*H*-pyrazole-3-carboxylic acid;
1-{1-[5-chloro-2-(2-chloro-4-fluoro-benzyloxy)-phenyl]-ethyl}-5-methyl-1H-pyrazole-3-carboxylic acid;
1-{5-chloro-2-[(2-fluorobenzyloxy)-phenyl]-ethyl}-5-methyl-1*H*-pyrazole-3-carboxylic acid;
1-{5-chloro-2-[(4-fluorobenzyloxy)-phenyl]-ethyl}-5-methyl-1*H*-pyrazole-3-carboxylic acid;
1-{5-chloro-2-[(2,4-difluorobenzyloxy)-phenyl]-ethyl}-5-methyl-1*H*-pyrazole-3-carboxylic acid;
1-{5-chloro-2-[(2,4,6-trifluorobenzyloxy)-phenyl]-ethyl}-5-methyl-1*H*-pyrazole-3-carboxylic acid;
1-{5-chloro-2-[(4-chloro-2-fluorobenzyloxy)-phenyl]-ethyl}-5-methyl-1*H*-pyrazole-3-carboxylic acid;
1-{5-chloro-2-[(benzyloxy)-phenyl]-ethyl}-5-methyl-1*H*-pyrazole-3-carboxylic acid;;
1-[1-(5-chloro-2-isobutoxy-phenyl)-ethyl]-5-methyl-1H-pyrazole-3-carboxylic acid;
1-[5-chloro-2-(2-fluoro-benzyloxy)-benzyl]-5-methyl-1H-pyrrole-3-carboxylic acid;
1-[5-chloro-2-{4-fluoro-benzyloxy)-benzyl]-5-methyl-1H-pyrrole-3-carboxylic acid;
1-[5-chloro-2-(2,4-difluoro-benzyloxy)benzyl]-5-methyl-1H-pyrrole-3-carboxylic acid;
1-[5-chloro-2-(4-chloro-2-fluoro-benzyloxy)-benzyl]-5-methyl-1H-pyrrole-3-carboxylic acid; and
1-[5-chloro-2-(2-chloro-4-fluoro-benzyloxy)-benzyl]-5-methyl-1H-pyrrole-3-carboxylic acid
and pharmaceutically acceptable salts or solvates thereof.

3. A pharmaceutical composition comprising a compound according to claim 1 or claim 2 or a pharmaceutically acceptable salt or solvate thereof together with a pharmaceutical carrier and/or excipient.

4. A compound according to claim 1 or claim 2 or a pharmaceutically acceptable salt or solvate thereof for use as an active therapeutic substance.

5. Use of a compound according to claim 1 or claim 2 or a pharmaceutically acceptable salt or solvate thereof for the manufacture of a medicament for the treatment or prevention of a condition such as pain, or an inflammatory, immunological, bone, neurodegenerative or renal disorder.

6. Use of a compound according to claim 1 or claim 2 or a pharmaceutically acceptable salt or solvate thereof for the manufacture of a medicament for the treatment or prevention of a condition such as inflammatory pain, neuropathic pain or visceral pain.

## Patentansprüche

1. Verbindung der Formel (I): 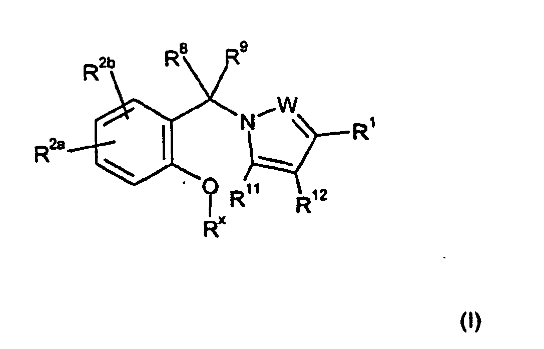 oder ein pharmazeutisch verträgliches Salz oder Solvat davon
wobei:
W N oder CR¹⁰ ist;
R¹ CO₂H ist;
R^{2a} und R^{2b} unabhängig ausgewählt sind aus einem Wasserstoffatom, einem Halogenatom, C₁₋₄-Alkyl, CF₃ und OC₁₋₆-Alkyl;
R^{x} ausgewählt ist aus CH₂-Pyridyl, C₁₋₆-Alkyl oder CH₂Ph, wobei Ph mit R^{3a}, R^{3b} und R^{3c} substituiert ist;
R^{3a}, R^{3b} und R^{3c} unabhängig ausgewählt sind aus einem Wasserstoffatom, einem Halogenatom, NO₂, OCH₃, CH₃ und CF₃;
R⁸ ein Wasserstoffatom ist;
R⁹ ein Wasserstoffatom oder CH₃ ist;
R¹⁰ ausgewählt ist aus einem Wasserstoffatom, einem Halogenatom, CH₃ und CF₃;
R¹¹ ausgewählt ist aus einem Wasserstoffatom, einem Halogenatom, CH₃, CF₃ und Thienyl; und
R¹² ausgewählt ist aus einem Wasserstoffatom, einem Halogenatom, CH₃ und CF₃.

2. Verbindung gemäß Anspruch 1, ausgewählt aus:
1-({5-Brom-2-[(phenylmethyl)oxy]phenyl}methyl)-5-methyl-1*H*-pyrazol-3-carbonsäure;
1-({5-Chlor-2-[(phenylmethyl)oxy]phenyl}methyl)-1H-pyrazol-3-carbonsäure;
1-[(5-Brom-2-{[(2,4-difluorphenyl)methyl]oxy}phenyl)methyl]-5-methyl-1*H*-pyrazol-3-carbonsäure;
1-({5-Chlor-2-[(2-methylpropyl)oxy]phenyl}methyl)-5-methyl-1H-pyrazol-3-carbonsäure;
1-{5-Brom-2-[(2,4-dichlorbenzyl)oxy]benzyl}-5-methyl-1*H*-pyrazol-3-carbonsäure;
1-{5-Brom-2-[(4-chlorbenzyl)oxy]benzyl}-5-methyl-1*H*-pyrazol-3-carbonsäure;
1-{5-Brom-2-[(4-fluorbenzyl)oxy]benzyl}-5-methyl-1H-pyrazol-3-carbonsäure;
1-{5-Brom-2-[(2-chlorbenzyl)oxy]benzyl}-5-methyl-1H-pyrazol-3-carbonsäure;
1-[2-(Benzyloxy)-5-brombenzyl]-1*H*-pyrazol-3-carbonsäure;
1-{5-Brom-2-[(2-methoxybenzyl)oxy]benzyl}-5-methyl-1*H*-pyrazol-3-carbonsäure;
1-(5-Brom-2-butoxybenzyl)-5-methyl-1*H*-pyrazol-3-carbonsäure;
1-(5-Brom-2-{[4-(trifluormethyl)benzyl]oxy}benzyl)-5-methyl-1*H*-pyrazol-3-carbonsäure;
1-{5-Brom-2-[(2,6-difluorbenzyl)oxy]benzyl}-5-methyl-1*H*-pyrazol-3-carbonsäure;
1-{5-Brom-2-[(3-brombenzyl)oxy]benzyl}-5-methyl-1*H*-pyrazol-3-carbonsäure;
1-{5-Brom-2-[(3-chlorbenzyl)oxy]benzyl}-5-methyl-1*H*-pyrazol-3-carbonsäure;
1-[5-Brom-2-(pyridin-4-ylmethoxy)benzyl]-5-methyl-1*H*-pyrazol-3-carbonsäure;
1-{5-Brom-2-[(3-methylbenzyl)oxy]benzyl}-5-methyl-1*H*-pyrazol-3-carbonsäure;
1-{5-Brom-2-[(3-nitrobenzyl)oxy]benzy1}-5-methyl-1*H*-pyrazol-3-carbonsäure;
1-[2-(Benzyloxy)-5-brombenzyl]-5-thien-2-yl-1*H*-pyrazol-3-carbonsäure;
1-[2-(Benzyloxy)-5-brombenzyl]-4-fluor-1*H*-pyrazol-3-carbonsäure;
1-[(5-Chlor-2-{[(4-fluorphenyl)methyl]oxy}phenyl)methyl]-5-methyl-1*H*-pyrazol-3-carbonsäure;
1-[(5-Chlor-2-{[(4-chlorphenyl)methyl]oxy}phenyl)methyl]-5-methyl-1*H*-pyrazol-3-carbonsäure;
1-[(5-Chlor-2-{[(2-chlorphenyl)methyl]oxy}phenyl)methyl]-5-methyl-1*H*-pyrazol-3-carbonsäure;
1-[(5-Chlor-2-{[(2,4-dichlorphenyl)methyl]oxy}phenyl)methyl]-5-methyl-1*H*-pyrazol-3-carbonsäure;
1-[(5-Chlor-2-{[(2,6-difluorphenyl)methyl]oxy}phenyl)methyl]-5-methyl-1H-pyrazol-3-carbonsäure;
1-[(5-Chlor-2-{[(2,4-difluorphenyl)methyl]oxy}phenyl)methyl]-5-methyl-1*H*-pyrazol-3-carbonsäure;
1-({5-Chlor-2-[(phenylmethyl)oxy]phenyl}methyl)-5-methyl-1*H*-pyrazol-3-carbonsäure;
1-{[2-{[(4-Fluorphenyl)methyl]oxy}-5-(methyloxy)phenyl]methyl}-5-methyl-1*H*-pyrazol-3-carbonsäure;
1-{[2-{[(4-Chlorphenyl)methyl]oxy}-5-(methyloxy)phenyl]methyl}-5-methyl-1*H*-pyrazol-3-carbonsäure;
1-{[2-{[(2-Chlorphenyl)methyl]oxy}-5-(methyloxy)phenyl]methyl}-5-methyl-1*H*-pyrazol-3-carbonsäure;
1-{[2-{[(2,4-Dichlorphenyl)methyl]oxy}-5-(methyloxy)phenyl]methyl}-5-methyl-1*H-*pyrazol-3-carbonsäure;
1-{[2-{[(2,6-Difluorphenyl)methyl]oxy}-5-(methyloxy)phenyl]methyl}-5-methyl-1*H-*pyrazol-3-carbonsäure;
1-{[2-{[(2,4-Difluorphenyl)methyl]oxy}-5-(methyloxy)phenyl]methyl}-5-methyl-1*H-*pyrazol-3-carbonsäure;
5-Methyl-1-({5-(methyloxy)-2-[(phenylmethyl)oxy]phenyl}methyl)-1*H*-pyrazol-3-carbonsäure;
1-[(2-{[(4-Fluorphenyl)methyl]oxy}phenyl)methyl]-5-methyl-1*H*-pyrazol-3-carbonsäure;
1-[(2-{[(4-Chlorpheny1)methyl]oxy}phenyl)methyl]-5-methyl-1*H*-pyrazol-3-carbonsäure;
1-[(2-([(2-Chlorphenyl)methyl]oxy)phenyl)methyl]-5-methyl-1*H*-pyrazol-3-carbonsäure;
1-[(2-([(2,4-Dichlorphenyl)methyl]oxy)phenyl)methyl]-5-methyl-1*H*-pyrazol-3-carbonsäure;
1-[(2-{[(2,6-Difluorphenyl)methyl]oxy}phenyl)methyl]-5-methyl-1*H*-pyrazol-3-carbonsäure;
1-[(2-{[(2,4-Difluorphenyl)methyl]oxy}phenyl)methyl]-5-methyl-1*H*-pyrazol-3-carbonsäure;
5-Methyl-1-({2-[(phenylmethyl)oxy]phenyllmethyl)-1*H*-pyrazol-3-carbonsäure;
1-[(5-Fluor-2-{[(4-fluorphenyl)methyl]oxy}phenyl)methyl]-5-methyl-1*H*-pyrazol-3-carbonsäure;
1-[(2-{[(4-Chlorphenyl)methyl]oxy}-5-fluorphenyl)methyl]-5-methyl-1*H*-pyrazol-3-carbonsäure;
1-[(2-{[(2-Chlorphenyl)methyl]oxy}-5-fluorphenyl)methyl]-5-methyl-1*H*-pyrazol-3-carbonsäure;
1-[(2-{[(2,4-Dichlorphenyl)methyl]oxy}-5-fluorphenyl)methyl]-5-methyl-1*H*-pyrazol-3-carbonsäure;
1-[(2-{[(2,6-Difluorphenyl)methyl]oxy}-5-fluorphenyl)methyl]-5-methyl-1*H*-pyrazol-3-carbonsäure;
1-[(2-{[(2,4-Difluorphenyl)methyl]oxy}-5-fluorphenyl)methyl]-5-methyl-1*H*-pyrazol-3-carbonsäure;
1-({5-Fluor-2-[(phenylmethyl)oxy]phenyl}methyl)-5-methyl-1*H*-pyrazol-3-carbonsäure;
1-{1-[5-Chlor-2-(2-chlor-4-fluorbenzyloxy)-phenyl]-ethyl}-5-methyl-1*H*-pyrazol-3-carbonsäure;
1-{5-Chlor-2-[(2-fluorbenzyloxy)-phenyl]-ethyl}-5-methyl-1*H*-pyrazol-3-carbonsäure;
1-{5-Chlor-2-[(4-fluorbenzyloxy)-phenyl]-ethyl}-5-methyl-1*H*-pyrazol-3-carbonsäure;
1-{5-Chlor-2-[(2,4-difluorbenzyloxy)-phenyl]-ethyl}-5-methyl-1*H*-pyrazol-3-carbonsäure;
1-{5-Chlor-2-[(2,4,6-trifluorbenzyloxy)-phenyl]-ethyl}-5-methyl-1*H*-pyrazol-3-carbonsäure;
1-}5-Chlor-2-[(4-chlor-2-fluorbenzyloxy)-phenyl]-ethyl}-5-methyl-1*H*-pyrazol-3-carbonsäure;
1-{5-Chlor-2-[(benzyloxy)-phenyl]-ethyl}-5-methyl-1*H*-pyrazol-3-carbonsäure;
1-[1-(5-Chlor-2-isobutoxyphenyl)-ethyl]-5-methyl-1*H*-pyrazol-3-carbonsäure;
1-[5-Chlor-2-(2-fluorbenzyloxy)-benzyl]-5-methyl-1*H*-pyrrol-3-carbonsäure;
1-[5-Chlor-2-(4-fluorbenzyloxy)-benzyl]-5-methyl-1*H*-pyrrol-3-carbonsäure;
1-[5-Chlor-2-(2,4-difluorbenzyloxy)-benzyl]-5-methyl-1*H*-pyrrol-3-carbonsäure;
1-[5-Chlor-2-(4-chlor-2-fluorbenzyloxy)-benzyl]-5-methyl-1*H*-pyrrol-3-carbonsäure; und
1-[5-Chlor-2-(2-chlor-4-fluorbenzyloxy)-benzyl]-5-methyl-1*H*-pyrrol-3-carbonsäure;
und pharmazeutisch verträgliche Salze oder Solvate davon.

3. Arzneimittel, umfassend eine Verbindung gemäß Anspruch 1 oder 2 oder ein pharmazeutisch verträgliches Salz oder Solvat davon zusammen mit einem pharmazeutischen Träger und/oder Exzipienten.

4. Verbindung gemäß Anspruch 1 oder 2 oder ein pharmazeutisch verträgliches Salz oder Solvat davon zur Verwendung als therapeutischer Wirkstoff.

5. Verwendung einer Verbindung gemäß Anspruch 1 oder 2 oder eines pharmazeutisch verträglichen Salzes oder Solvats davon zur Herstellung eines Medikaments zur Behandlung oder Vorbeugung eines Zustands wie Schmerz oder einer entzündlichen, immunologischen, Knochen-, neurodegenerativen oder renalen Störung.

6. Verwendung einer Verbindung gemäß Anspruch 1 oder 2 oder eines pharmazeutisch verträglichen Salzes oder Solvats davon zur Herstellung eines Medikaments zur Behandlung oder Vorbeugung eines Zustands wie entzündlicher Schmerz, neuropathischer Schmerz oder viszeraler Schmerz.

## Revendications

1. Composé de formule (I) : 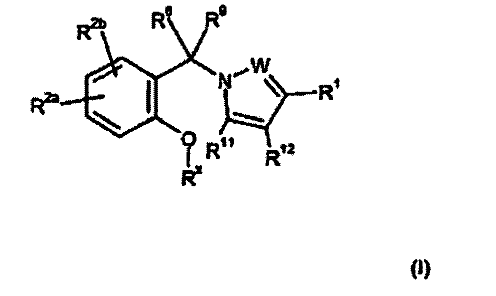 dans lequel
W représente N ou un groupe CR¹⁰ ;
R¹ représente un groupe CO₂H ;
R^{2a} et R^{2b} sont choisis indépendamment entre un atome d'hydrogène, des groupes halogéno, alkyle en C₁ à C₄, CF₃ et O(alkyle en C₁ à C₆) ;
R^{x} est choisi entre des groupes CH₂-pyridyle, alkyle en C₁ à C₆ et CH₂Ph dans lequel Ph est substitué avec des groupes R^{3a}, R^{3b} et R^{3c} ;
R^{3a}, R^{3b} et R^{3c} sont choisis indépendamment entre un atome d'hydrogène, un atome d'halogène, des groupes NO₂, OCH₃, CH₃ et CF₃ ;
R⁸ représente un atome d'hydrogène ;
R⁹ représente un atome d'hydrogène ou un groupe CH₃ ;
R¹⁰ est choisi entre un atome d'hydrogène, un atome d'halogène, des groupes CH₃ et CF₃ ;
R¹¹ est choisi entre un atome d'hydrogène, un atome d'halogène, des groupes CH₃, CF₃ et thiényle ; et
R¹² est choisi entre un atome d'hydrogène, un atome d'halogène, des groupes CH₃ et CF₃ ;
ou un de ses sels ou produits de solvatation pharmaceutiquement acceptables.

2. Composé suivant la revendication 1, choisi entre :
l'acide 1-({5-bromo-2-[(phénylméthyl)oxy]phényl}méthyl)-5-méthyl-1*H*-pyrazole-3-carboxylique ;
l'acide 1-({5-chloro-2-[(phénylméthyl)oxy]phényl}méthyl)-1*H*-pyrazole-3-carboxylique ;
l'acide 1-[(5-bromo-2-{[(2,4-difluorophényl)méthyl]oxy}-phényl)méthyl]-5-méthyl-1*H*-pyrazole-3-carboxylique ;
l'acide 1-({5-chloro-2-[(2-méthylpropyl)oxy]phényl}méthyl)-5-méthyl-1*H*-pyrazole-3-carboxylique ;
l'acide 1-{5-bromo-2-[(2,4-dichlorobenzyl)oxy]benzyl}-5-méthyl-1H-pyrazole-3-carboxylique ;
l'acide 1-{5-bromo-2-[(4-chlorobenzyl)oxy]benzyl}-5-méthyl-1H-pyrazole-3-carboxylique ;
l'acide 1-{5-bromo-2-[(4-fluorobenzyl)oxy]benzyl}-5-méthyl-1H-pyrazole-3-carboxylique ;
l'acide 1-{5-bromo-2-[(2-chlorobenzyl)oxy]benzyl}-5-méthyl-1H-pyrazole-3-carboxylique ;
l'acide 1-[2-(benzyloxy)-5-bromobenzyl]-1H-pyrazole-3-carboxylique ;
l'acide 1-{5-bromo-2-[(2-méthoxybenzyl)oxy]benzyl}-5-méthyl-1H-pyrazole-3-carboxylique ;
l'acide 1-(5-bromo-2-butoxybenzyl)-5-méthyl-1H-pyrazole-3-carboxylique ;
l'acide 1-(5-bromo-2-{[4-(trifluorométhyl)benzyl]oxy}-benzyl)-5-méthyl-1H-pyrazole-3-carboxylique ;
l'acide 1-{5-bromo-2-[(2,6-difluorobenzyl)oxy]benzyl}-5-méthyl-1H-pyrazole-3-carboxylique ;
l'acide 1-{5-bromo-2-[(3-bromobenzyl)oxy]benzyl}-5-méthyl-1H-pyrazole-3-carboxylique ;
l'acide 1-{5-bromo-2-[(3-chlorobenzyl)oxy]benzyl}-5-méthyl-1H-pyrazole-3-carboxylique ;
l'acide 1-[5-bromo-2-(pyridine-4-ylméthoxy)benzyl]-5-méthyl-1H-pyrazole-3-carboxylique ;
l'acide 1-{5-bromo-2-[(3-méthylbenzyl)oxy]benzyl}-5-méthyl-1H-pyrazole-3-carboxylique ;
l'acide 1-{5-bromo-2-[(3-nitrobenzyl)oxy]benzyl}-5-méthyl-1H-pyrazole-3-carboxylique ;
l'acide 1-[2-(benzyloxy)-5-bromobenzyl]-5-thiène-2-yl-1H-pyrazole-3-carboxylique ;
l'acide 1-[2-(benzyloxy)-5-bromobenzyl]-4-fluoro-1H-pyrazole-3-carboxylique ;
l'acide 1-[(5-chloro-2-{[(4-fluorophényl)méthyl]oxy}-phényl)méthyl]-5-méthyl-1*H*-pyrazole-3-carboxylique ;
l'acide 1-[(5-chloro-2-{[(4-chlorophényl)méthyl]oxy}-phényl)méthyl]-5-méthyl-1*H*-pyrazole-3-carboxylique ;
l'acide 1-[(5-chloro-2-{[(2-chlorophényl)méthyl]oxy}-phényl)méthyl]-5-méthyl-1*H*-pyrazole-3-carboxylique ;
l'acide 1-[(5-chloro-2-[[(2,4-dichlorophényl)méthyl]oxy]-phényl)méthyl]-5-méthyl-1*H*-pyrazole-3-carboxylique ;
l'acide 1-[(5-chloro-2-{[(2,6-difluorophényl)méthyl]oxy}-phényl)méthyl]-5-méthyl-1*H*-pyrazole-3-carboxylique ;
l'acide 1-[(5-chloro-2-{[(2,4-difluorophényl)méthyl]oxy}-phényl)méthyl]-5-méthyl-1*H*-pyrazole-3-carboxylique ;
l'acide 1-({5-chloro-2-[(phénylméthyl)oxy]phényl}méthyl)-5-méthyl-1H-pyrazole-3-carboxylique ;
l'acide 1-{[2-{[(4-fluorophényl)méthyl]oxy}-5-(méthyloxy)-phényl]méthyl}-5-méthyl-1*H*-pyrazole-3-carboxylique ;
l'acide 1-{[2-{[(4-chlorophényl)méthyl]oxy}-5-(méthyloxy)-phényl]méthyl}-5-méthyl-1*H*-pyrazole-3-carboxylique ;
l'acide 1-{[2-{[(2-chlorophényl)méthyl]oxy}-5-(méthyloxy)-phényl]méthyl}-5-méthyl-1*H*-pyrazole-3-carboxylique ;
l'acide 1-{[2-{[(2,4-dichlorophényl)méthyl]oxy}-5-(méthyloxy)-phényl]méthyl}-5-méthyl-1*H*-pyrazole-3-carboxylique ;
l'acide 1-{[2-{[(2,6-difluorophényl)méthyl]oxy}-5-(méthyloxy)-phényl]méthyl}-5-méthyl-1*H*-pyrazole-3-carboxylique ;
l'acide 1-{[2-{[(2,4-difluorophényl)méthyl]oxy}-5-(méthyloxy)-phényl]méthyl}-5-méthyl-1*H*-pyrazole-3-carboxylique ;
l'acide 5-méthyl-1-({5-(méthyloxy)-2-[(phénylméthyl)oxy]-phényl}méthyl)-1*H*-pyrazole-3-carboxylique ;
l'acide 1-[(2-{[(4-fluorophényl)méthyl]oxy}phényl)méthyl]-5-méthyl-1*H*-pyrazole-3-carboxylique ;
l'acide 1-[(2-{[(4-chlorophényl)méthyl]oxy}phényl)méthyl]-5-méthyl-1*H*-pyrazole-3-carboxylique ;
l'acide 1-[(2-{[(2-chlorophényl)méthyl]oxy}phényl)méthyl]-5-méthyl-1*H*-pyrazole-3-carboxylique ;
l'acide 1-[(2-{[(2,4-dichlorophényl)méthyl]oxy)phényl)-méthyl]-5-méthyl-1*H*-pyrazole-3-carboxylique ;
l'acide 1-[(2-{[(2,6-difluorophényl)méthyl]oxy}phényl)-méthyl]-5-méthyl-1*H*-pyrazole-3-carboxylique ;
l'acide 1-[(2-{[(2,4-difluorophényl)méthyl]oxy}phényl)-méthyl]-5-méthyl-1*H*-pyrazole-3-carboxylique ;
l'acide 5-méthyl-1-({2-[(phénylméthyl)oxy]phényl}méthyl)-1*H*-pyrazole-3-carboxylique ;
l'acide 1-[(5-fluoro-2-{[(4-fluorophényl)méthyl]oxy}-phényl)méthyl]-5-méthyl-1*H*-pyrazole-3-carboxylique ;
l'acide 1-[(2-{[(4-chlorophényl)méthyl]oxy}-5-fluorophényl)-méthyl]-5-méthyl-1*H*-pyrazole-3-carboxylique ;
l'acide 1-[(2-{[(2-chlorophényl)méthyl]oxy}-5-fluorophényl)-méthyl]-5-méthyl-1H-pyrazole-3-carboxylique ;
l'acide 1-[(2-{[(2,4-dichlorophényl)méthyl]oxy}-5-fluorophényl)méthyl]-5-méthyl-1*H*-pyrazole-3-carboxylique ;
l'acide 1-[(2-{[(2,6-difluorophényl)méthyl]oxy}-5-fluorophényl)méthyl]-5-méthyl-1*H*-pyrazole-3-carboxylique ;
l'acide 1-[(2-{[(2,4-difluorophényl)méthyl]oxy}-5-fluorophényl)méthyl]-5-méthyl-1*H*-pyrazole-3-carboxylique ;
l'acide 1-({5-fluoro-2-[(phénylméthyl)oxy]phényl}méthyl)-5-méthyl-1H-pyrazole-3-carboxylique ;
l'acide 1-{1-[5-chloro-2-(2-chloro-4-fluoro-benzyloxy)-phényl]-éthyl}-5-méthyl-1*H*-pyrazole-3-carboxylique;
l'acide 1-{5-chloro-2-[(2-fluorobenzyloxy)-phényl]-éthyl}-5-méthyl-1*H*-pyrazole-3-carboxylique ;
l'acide 1-{5-chloro-2-[(4-fluorobenzyloxy)-phényl]-éthyl}-5-méthyl-1*H*-pyrazole-3-carboxylique ;
l'acide 1-{5-chloro-2-[(2,4-difluorobenzyloxy)-phényl]-éthyl)-5-méthyl-1*H*-pyrazole-3-carboxylique ;
l'acide 1-{5-chloro-2-[(2,4,6-trifluorobenzyloxy)-phényl]-éthyl}-5-méthyl-1*H*-pyrazole-3-carboxylique ;
l'acide 1-{5-chloro-2-[(4-chloro-2-fluorobenzyloxy)-phényl]-éthyl}-5-méthyl-1*H*-pyrazole-3-carboxylique ;
l'acide 1-{5-chloro-2-[(benzyloxy)-phényl]-éthyl}-5-méthyl-1*H*-pyrazole-3-carboxylique ;
l'acide 1-[1-(5-chloro-2-isobutoxy-phényl)-éthyl]-5-méthyl-1*H*-pyrazole-3-carboxylique ;
l'acide 1-[5-chloro-2-(2-fluoro-benzyloxy)-benzyl]-5-méthyl-1H-pyrrole-3-carboxylique ;
l'acide 1-[5-chloro-2-(4-fluoro-benzyloxy)-benzyl]-5-méthyl-1H-pyrrole-3-carboxylique ;
l'acide 1-[5-chloro-2-(2,4-difluoro-benzyloxy)-benzyl]-5-méthyl-1H-pyrrole-3-carboxylique ;
l'acide 1-[5-chloro-2-(4-chloro-2-fluoro-benzyloxy)-benzyl]-5-méthyl-1H-pyrrole-3-carboxylique ; et
l'acide 1-[5-chloro-2-(2-chloro-4-fluoro-benzyloxy)-benzyl]-5-méthyl-1H-pyrrole-3-carboxylique
et ses sels ou produits de solvatation pharmaceutiquement acceptables.

3. Composition pharmaceutique comprenant un composé suivant la revendication 1 ou la revendication 2 ou un de ses sels ou produits de solvatation pharmaceutiquement acceptables conjointement avec un support et/ou excipient pharmaceutique.

4. Composé suivant la revendication 1 ou la revendication 2 ou un de ses sels ou produits de solvatation pharmaceutiquement acceptables, destiné à être utilisé comme substance thérapeutique active.

5. Utilisation d'un composé suivant la revendication 1 ou la revendication 2 ou d'un de ses sels ou produits de solvatation pharmaceutiquement acceptables pour la production d'un médicament destiné au traitement ou à la prévention d'une affection telle que la douleur, ou d'un trouble inflammatoire, immunologique, osseux, neurodégénératif ou rénal.

6. Utilisation d'un composé suivant la revendication 1 ou la revendication 2 ou d'un de ses sels ou produits de solvatation pharmaceutiquement acceptables pour la production d'un médicament destiné au traitement ou à la prévention d'une affection telle que la douleur inflammatoire, la douleur neuropathique ou la douleur viscérale.
